(19) Europäisches Patentamt
European Patent Office
Office européen des brevets

(11) Veröffentlichungsnummer: **0 452 712 B1**

(12) **EUROPÄISCHE PATENTSCHRIFT**

(45) Veröffentlichungstag der Patentschrift: **19.04.95**

(21) Anmeldenummer: **91104723.1**

(22) Anmeldetag: **26.03.91**

Die Akte enthält technische Angaben, die nach dem Eingang der Anmeldung eingereicht wurden und die nicht in dieser Patentschrift enthalten sind.

(51) Int. Cl.6: **C07D 401/04**, C07D 401/14, C07D 409/14, C07D 491/04, C07D 409/04, C07D 215/14, C07D 217/04, A61K 31/47

# (54) Neue 4-Chinolyl-dihydropyridine, Verfahren zu ihrer Herstellung und ihre Verwendung in Arzneimitteln.

(30) Priorität: **06.04.90 DE 4011105**

(43) Veröffentlichungstag der Anmeldung: **23.10.91 Patentblatt 91/43**

(45) Bekanntmachung des Hinweises auf die Patenterteilung: **19.04.95 Patentblatt 95/16**

(84) Benannte Vertragsstaaten: **AT BE CH DE DK ES FR GB GR IT LI LU NL SE**

(56) Entgegenhaltungen:
**EP-A- 0 002 208**
**EP-A- 0 123 095**
**EP-A- 0 123 112**
**DE-A- 2 117 571**
**DE-A- 2 210 667**
**DE-A- 2 658 804**
**DE-A- 3 447 169**

**JOURNAL OF MEDICINAL CHEMISTRY vol. 13, no. 5, September 1970, Seiten 860 -864; J.S. GILLESPIE JR ET AL: "Antimalarials. II. 8-Ouinolinemethanols"**

(73) Patentinhaber: **BAYER AG**

**D-51368 Leverkusen (DE)**

(72) Erfinder: **Stoltefuss, Jürgen, D.I.**
**Parkstrasse 20**
**W-5657 Haan 2 (DE)**
Erfinder: **Böshagen, Horst, Dr.**
**Wiesenstrasse 4**
**W-5657 Haan (DE)**
Erfinder: **Goldmann, Siegfried, Dr.**
**Am Osterholz 91**
**W-5600 Wuppertal 11 (DE)**
Erfinder: **Straub, Alexander, Dr.**
**Moospfad 30**
**W-5600 Wuppertal (DE)**
Erfinder: **Gross, Rainer, Prof. Dr.**
**Platzhoffstrasse 23**
**W-5600 Wuppertal (DE)**
Erfinder: **Hütter, Joachim, Dr.**
**Weinhauserstrasse 9**
**W-5090 Leverkusen 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**JOURNAL OF HETEROCYCLIC CHEMISTRY vol. 6, no. 2, April 1969, Seiten 243 - 245;J.B. WOMMACK ET AL: "The synthesis of quinoli- ne- andisoquinolinecarboxaldehydes (1)"**

Erfinder: **Hebisch, Siegbert, Dr.**
**Richard-Seel-Weg 11**
**W-5600 Wuppertal 1 (DE)**
Erfinder: **Bechem, Martin, Dr.**
**Hans-Böckler-Strasse 102**
**W-5600 Wuppertal 1 (DE)**

## Beschreibung

Die Erfindung betrifft neue 4-Chinolyl-dihydropyridine, Verfahren zu ihrer Herstellung und ihre Verwendungin Arzneimitteln, insbesondere in Mitteln mit positiv inotroper Wirkung.

Es ist bereits bekannt, daß 1,4-Dihydropyridine gefäßerweiternde Eigenschaften besitzen und als Coronarmittel und Antihypertensiva verwendet werden können (vgl. GB-A-1 358 951; DE-A-2 629 892 und DE-A-27 52 820). Weiterhin ist bekannt, daß 1,4-Dihydropyridine eine Hemmung der Kontraktionskraft von glatten und cardialen Muskeln bewirken und zur Behandlung von Coronar- und Gefäßerkrankungen eingesetzt werden können (vgl. Fleckenstein, Ann. Rev. Pharmacol. Toxicol., 17, 149 - 166 (1977)).

In DE-A-2 210 667 und DE-A-2 117 571 werden 1,4-Dihydropyridine beschrieben, die 2 Carboxylgruppen tragen während die erfindungsgemäßen Verbindungen nur eine Carboxylfunktion aufweisen. In EP-A-2 208 und DE-A-2 658 804 werden bereits Dihydropyridine beschrieben mit Chinolin- und Isochinolinresten in 4-Position, welche jedoch im Gegensatz zu den erfindungsgemäßen Verbindungen einen aromatischen Rest als Substituenten tragen. In EP-A-123 112 und EP-A-123 095 werden Chromon- und Thiochromonderivate mit positiv inotroper Wirkung offenbart.

Es ist außerdem bekannt, daß 3-Nitro-dihydropyridine im allgemeinen neben einer positiv inotropen Herzwirkung den Nachteil einer unerwünschten konstringierenden Wirkung an den Koronargefäßen zeigen können (vgl. Schramm et al., Nature 303, 535-537 (1983) und DE-A-3 447 169).

Die Erfindung betrifft neue substituierte Dihydropyridine der allgemeinen Formel (I),

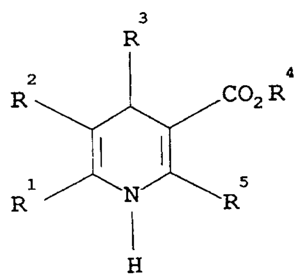

(I)

in welcher

$R^1$ und $R^5$ gleich oder verschieden sind und für geradkettiges oder verzweigtes Alkyl mit bis zu 6 Kohlenstoffatomen stehen,

$R^2$ für Nitro oder Cyano steht, oder

$R^1$ und $R^2$ gemeinsam einen Lactonring der Formel

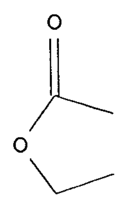

bilden,

$R^3$ für einen Rest der Formel

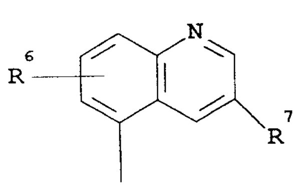

oder

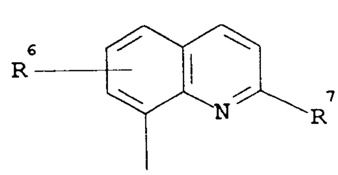

steht, worin

$R^6$ Wasserstoff, Fluor, Chlor oder geradkettiges oder verzweigtes Alkyl oder Alkoxy mit jeweils bis zu 2 Kohlenstoffatomen bedeutet,

$R^7$ Phenyl bedeutet, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen substituiert ist, oder Thienyl oder Pyridyl bedeutet, die gegebenenfalls durch Fluor oder Chlor monosubstituiert sind,

$R^4$ für Wasserstoff oder
für geradkettiges oder verzweigtes Alkyl oder Alkenyl mit jeweils bis zu 8 Kohlenstoffatomen steht, die gegebenenfalls durch Fluor, Chlor, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 6 Kohlenstoffatomen oder durch Phenoxy oder Phenyl substituiert sind

und deren physiologisch unbedenklichen Salze.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure oder Methansulfonsäure, Ethansulfonsäure, Phenylsulfonsäure, Toluolsulfonsäure oder Naphthalidinsulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E. L. Eliel, Stereochemistry of Carbon Compounds, McGraw Hill, 1962).

Besonders bevorzugt sind Verbindungen der allgemeinen Formel (I), in welcher

$R^1$ und $R^5$ gleich oder verschieden sind und für geradkettiges oder verzweigtes Alkyl mit bis zu 6 Kohlenstoffatomen stehen,

$R^2$ für Nitro oder Cyano steht, oder

$R^1$ und $R^2$ gemeinsam einen Lactonring der Formel

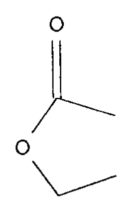

bilden,

$R^3$ für einen Rest der Formel

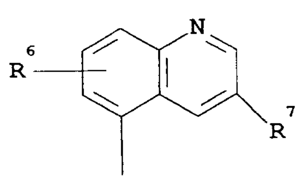

oder

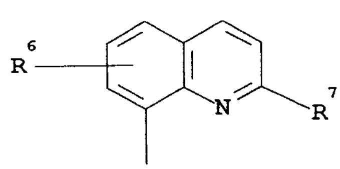

steht,
worin

$R^6$ Wasserstoff, Chlor oder Methyl bedeutet,

$R^7$ Phenyl bedeutet, das gegebenenfalls durch Fluor, Chlor, Nitro, Trifluormethyl oder durch geradkettiges oder verzweigtes Alkyl oder Alkoxy mit jeweils bis zu 4 Kohlenstoffatomen substituiert ist, oder
Pyridyl oder Thienyl bedeutet, die gegebenenfalls durch Fluor oder Chlor monosubstituiert wird,

$R^4$ für Wasserstoff steht, oder
für geradkettiges oder verzweigtes Alkyl mit bis zu 6 Kohlenstoffatomen steht, das gegebenenfalls durch Hydroxy, Carboxy, Cyano oder durch geradkettiges oder verzweigtes Alkoxycarbonyl, Alkoxy oder Acyloxy mit jeweils bis zu 4 Kohlenstoffatomen substituiert ist,

und deren physiologisch unbedenklichen Salze.

Außerdem wurde ein Verfahren zur Herstellung der erfindungsgemäßen Verbindungen der allgemeinen Formel (I) gefunden, dadurch gekennzeichnet, daß man im Fall, daß $R^1$ und $R^2$ die oben angegebene Bedeutung haben, aber nicht gemeinsam einen Lactonring bilden,

[A] Verbindungen der allgemeinen Formel (II)

$R^3$—CHO (II)

in welcher $R^3$ die oben angegebene Bedeutung hat,
zunächst mit Acetessigestern der allgemeinen Formel (III)

$R^5$-CO-$CH_2$-$CO_2$-$R^4$ (III)

in welcher
$R^4$ und $R^5$ die oben angegebene Bedeutung haben,
gegebenenfalls unter Isolierung der entsprechenden Ylidenverbindungen der allgemeinen Formel (IV)

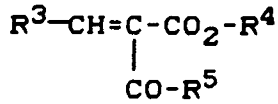 (IV)

in welcher
$R^3$, $R^4$ und $R^5$ die oben angegebene Bedeutung haben,
umsetzt und anschließend
entweder mit Verbindungen der Formel (V)

$R^1$-CO-$CH_2$-$R^2$ (V)

in welcher
$R^1$ und $R^2$ die oben angegebene Bedeutung haben,
in Anwesenheit von Ammoniak oder Ammoniumsalzen, oder direkt mit Aminoderivaten der allgemeinen Formel (VI)

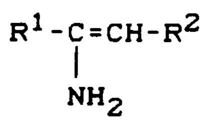 (VI)

in welcher
$R^1$ und $R^2$ die oben angegebene Bedeutung haben,
gegebenenfalls in Gegenwart von inerten organischen Lösemitteln umsetzt, oder

[B] die Aldehyde der allgemeinen Formel (II) zunächst mit den Verbindungen der allgemeinen Formel (V), gegebenenfalls unter Isolierung der Ylidenverbindungen der allgemeinen Formel (VII)

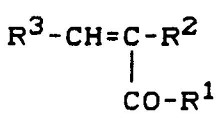 (VII)

in welcher
$R^1$, $R^2$ und $R^3$ die oben angegebene Bedeutung haben,
umsetzt und in einem nächsten Schritt mit den oben aufgeführten Verbindungen der allgemeinen

Formel (III) in inerten Lösemitteln, in Anwesenheit von Ammoniak oder Ammoniumsalzen oder direkt mit Enaminocarbonsäurederivaten der allgemeinen Formel (VIII)

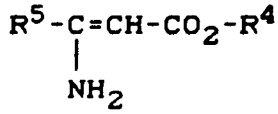

(VIII)

in welcher

$R^4$ und $R^5$ die oben angegebene Bedeutung haben,

umsetzt,

oder im Fall, daß $R^1$ und $R^2$ gemeinsam einen Lactonring bilden,

[C] zunächst nach denen unter [A] und [B] aufgeführten Methoden, Verbindungen der allgemeinen Formel (Ia)

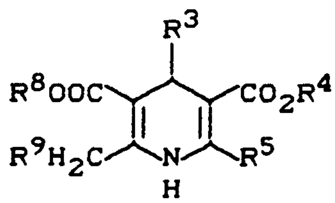

(Ia)

in welcher

$R^3$, $R^4$ und $R^5$ die oben angegebene Bedeutung haben,

$R^8$ für einen $C_1$-$C_6$-Alkyl-Rest steht

und

$R^9$ für eine Abgangsgruppe wie beispielsweise Chlor oder Acetoxy steht,

herstellt und nach bekannten Verfahren einen säure- oder basenkatalysierten Ringschluß anschließt,

und im Fall, daß $R^4$ nicht Wasserstoff bedeutet,

[D] Verbindungen der allgemeinen Formel (I), in welcher $R^1$, $R^2$, $R^3$, $R^4$ und $R^5$ die oben angegebene Bedeutung haben und $R^4$ für Wasserstoff steht, gegebenenfalls über ein reaktives Säurederivat, mit den entsprechenden Alkoholen umsetzt, wobei durch Einsatz der enantiomerenreinen Carbonsäuren ($R^4$ = H) die entsprechenden Enantiomere der Ester erhalten werden.

Die erfindungsgemäßen Verfahren können durch folgendes Formelschema erläutert werden:

[A]

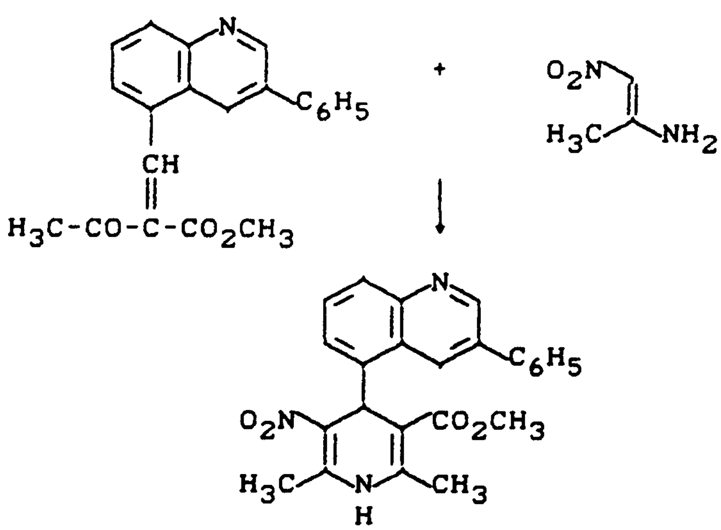

[B]

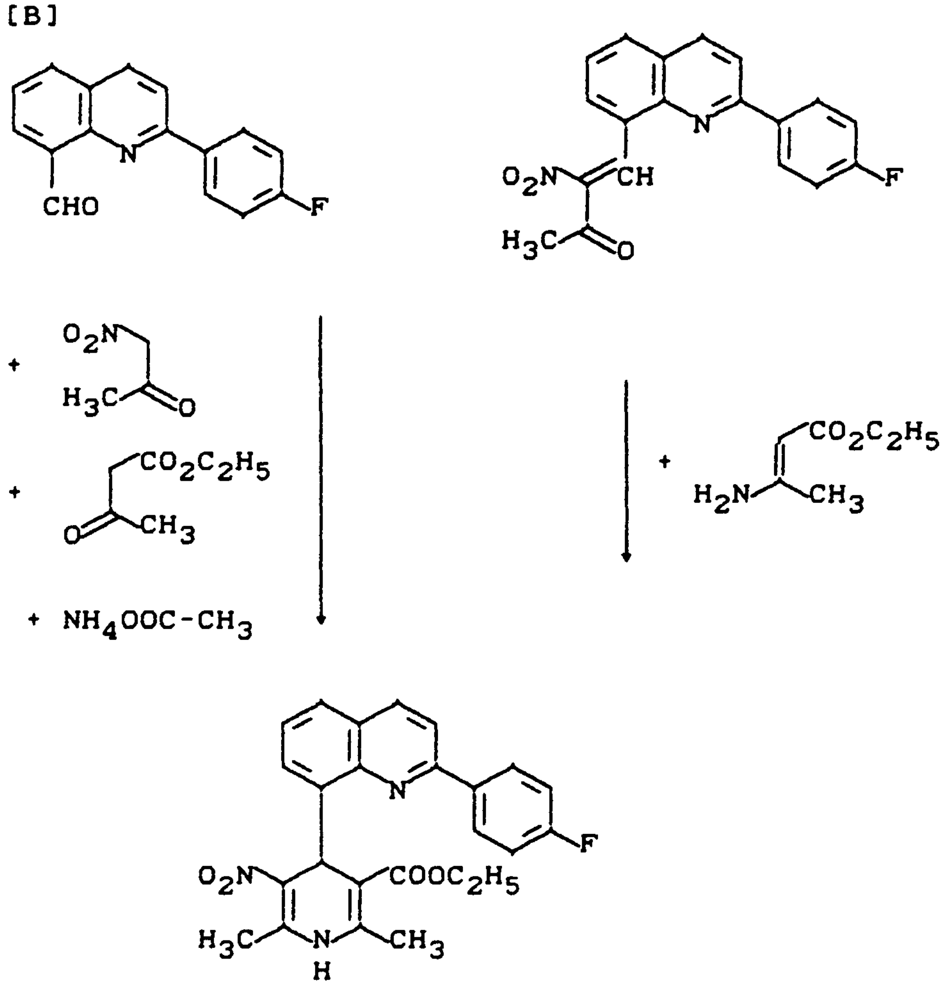


[C]

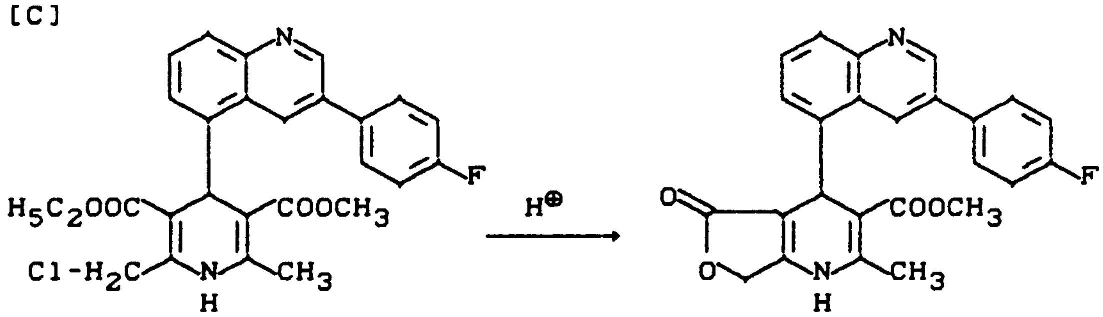


[D]

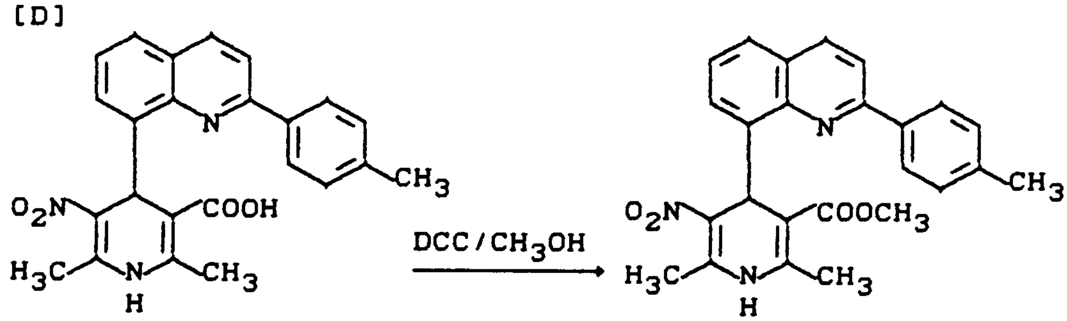

Als Lösemittel eignen sich für die Verfahren [A], [B] und [C] alle inerten organischen Lösemittel. Hierzu gehören bevorzugt Alkohole wie Methanol, Ethanol, n- bzw. iso-Propanol, Ether wie Diethylether, Tetrahydrofuran, Dioxan oder Glykolmono- oder dimethylether, Eisessig, Pyridin, Dimethylformamid, Dimethylsulfoxid, Acetonitril oder Hexamethylphosphorsäuretriamid oder Toluol.

Als Lösemittel für das Verfahren [D] eignen sich die oben aufgeführten Lösemittel mit Ausnahme der Alkohole.

Die Reaktionstemperatur für die Verfahren [A], [B], [C] und [D] können in einem größeren Bereich variiert werden. Im allgemeinen arbeitet man in einem Bereich von 10° C bis 200° C, bevorzugt von 20° C bis 150° C.

Die Verfahren können bei Normaldruck, erhöhtem oder erniedrigtem Druck (beispielsweise von 0,5 bis 5 bar), vorzugsweise bei Normaldruck durchgeführt werden.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Zur Aktivierung der Carbonsäure eignen sich die üblichen Reagenzien wie anorganische Halogenide, beispielsweise Thionylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Enantiomerenreine Formen erhält man z.B., indem man Diastereomerengemische der Verbindungen der allgemeinen Formel (I), in welcher $R^4$ für einen optischen Esterrest steht, nach üblicher Methode trennt, anschließend die enantiomerenreinen Carbonsäuren herstellt und dann gegebenenfalls durch Veresterung mit entsprechenden Alkoholen in die enantiomerenreinen Dihydropyridine überführt.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Veresterung der enantiomerenreinen Dihydropyridine erfolgt bevorzugt in Ethern wie Diethylether oder Tetrahydrofuran, Dimethylformamid, Methylchlorid, Chloroform, Acetonitril oder Toluol.

Die Aldehyde der allgemeinen Formel (II) sind ebenfalls neu und können hergestellt werden, indem man

a) entweder Verbindungen der allgemeinen Formel (IX)

$R^3$-$CH_3$ (IX)

in welcher

$R^3$ die oben angegebene Bedeutung hat,

direkt zu Verbindungen der Formel (II) oxidiert, oder zuerst zu Verbindungen der allgemeinen Formel (X)

$R^3$-$CH_2$-Hal (X)

in welcher

$R^3$ die oben angegebene Bedeutung hat

und

Hal für Halogen, vorzugsweise für Brom steht,

halogeniert, anschließend mit Acetationen umsetzt und zu Verbindungen der Formel (XI)

$R^3$-$CH_2$-OH (XI)

in welcher

$R^3$ die oben angegebene Bedeutung hat,

verseift und diese dann zu Verbindungen der Formel (II) oxidiert,

oder indem man

b) Verbindungen der allgemeinen Formel (XII),

$R^3$-COOR (XII)

in welcher

$R^3$ die oben angegebene Bedeutung hat

und

R

- für Wasserstoff oder einen $C_1$-$C_6$-Alkylrest steht,

entweder direkt reduziert oder durch Reduktion zunächst Verbindungen der Formel (XI) herstellt und diese dann oxidiert.

Das erfindungsgemäße Verfahren kann durch folgendes Formelschema beispielshaft erläutert werden:

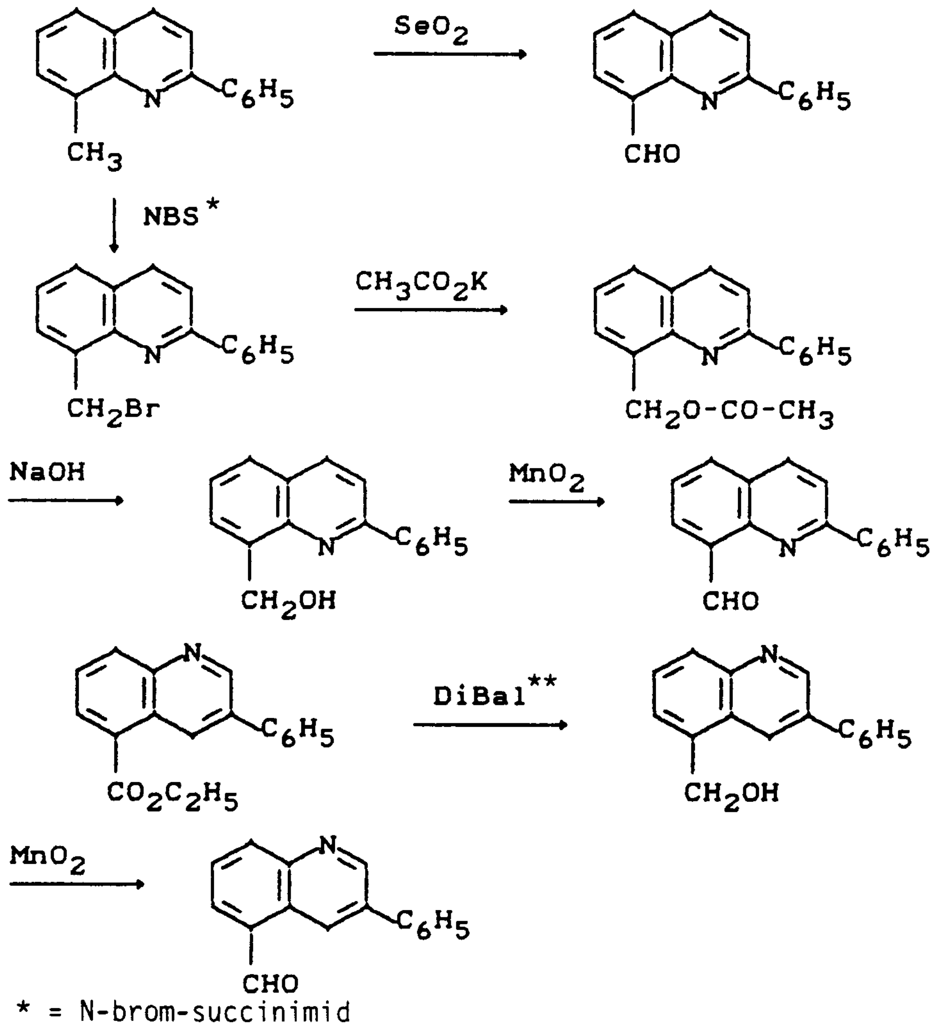


* = N-brom-succinimid

** = Di-iso-butyl-aluminiumhydrid

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure sowie Methylenchlorid, Tetrachlorkohlenstoff oder Toluol. Ebenso ist es möglich, Gemische der genannten Lösemittel zu verwenden.

Die Reduktion der Verbindungen der allgemeinen Formel (XII) erfolgt im allgemeinen mit Hydriden, bevorzugt mit Natriumborhydrid und Diisobutylaluminiumhydrid in inerten Lösemitteln wie Ethern, Kohlenwasserstoffen, Halogenkohlenwasserstoffen oder deren Gemische, bevorzugt in Ethern wie beispielsweise Diethylether, Tetrahydrofuran oder Dioxan.

Im Fall der Verbindungen der allgemeinen Formel (IX) eignen sich für die Oxidation beispielsweise Chromylchlorid, Cerammoniumnitrat, Silber(II)oxid, Selendioxid oder ein Chrom(VI)oxid in Verbindung mit

Essigsäureanhydrid. Bevorzugt ist Selendioxid.

Als Oxidationsmittel eignen sich im Fall der Hydroxymethyl-Verbindungen beispielsweise Mangandioxid, Dimethylsulfoxid, Cer-Ammoniumnitrat, Dipyridin-Chrom(VI)oxid, Natriumdichromat, Jodosobenzol, Pyridinchlorochromat, Silbercarbonat auf Celit oder Jones-Reagenz.

Die Oxidationen und Reduktionen können bei Normaldruck oder erhöhtem oder erniedrigtem Druck (beispielsweise von 0,5 bis 50 bar), vorzugsweise bei Normaldruck durchgeführt werden.

Die Verbindungen der allgemeinen Formel (IX) sind bekannt oder können nach herkömmlichen Methoden hergestellt werden [vgl. J. Med. Chem. 32, 396-401 (1989)].

Die Verbindungen der allgemeinenen Formel (XII) sind z.T. bekannt (R ≠ H) [vgl. aber J. Med. Chem. 16, 118-122 (1973), J. Med. Chem. 31, 1048-1052 (1988) und Monatssch. Chem., 114 (8-9), 1009-11] können aber nach bekannten Methoden hergestellt werden.

Die Verbindungen der allgemeinen Formel (XII) sind im Fall, daß R für Wasserstoff steht und $R^3$ den Rest der Formel

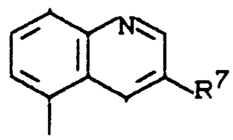

bedeutet neu und können dann über ein neuartiges Verfahren hergestellt werden, indem man 4-Nitro-3-hydroxyphthalid der Formel (XIII)

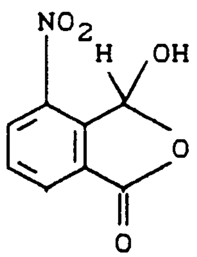

(XIII)

zunächst in inerten Lösungsmitteln, bevorzugt durch Hydrierung in Anwesenheit eines Katalysators zu 4-Amino-3-hydroxyphthalid der Formel (XIV)

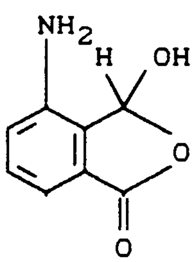

(XIV)

reduziert, gegebenenfalls isoliert, oder vorzugsweise direkt in Lösung mit Aldehyden der allgemeinen Formel (XV)

$R^7-CH_2-CHO$ (XV)

in welcher

$R^7$ die oben angegebene Bedeutung hat, in inerten Lösungsmitteln, gegebenenfalls in Anwesenheit einer Base umsetzt.

Als Lösemittel eignen sich sowohl für die Hydrierung als auch für die weitere Umsetzung alle organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylglykoldimethylether oder Amide wie Hexamethylphosphorsäuretriamid oder Dimethylformamid oder Essigsäure sowie Methylenchlorid, Tetrachlorkohlenstoff oder Toluol. Ebenso ist es möglich, Gemische der genannten Lösemitel zu verwenden. Bevorzugt sind Methanol, Ethanol, Propanol oder Tetrahydrofuran.

Die Hydrierung kann bei Normaldruck oder bei erhöhtem Druck, beispielsweise von 0,5 bis 5 bar, vorzugsweise bei Atmosphärendruck durchgeführt werden.

Die Reduktion erfolgt in einem Temperturbereich von 0° C bis 80° C, vorzugsweise bei Raumtemperatur.

Als Katalysator eignet sich insbesondere Palladium/Bariumsulfat. Es können aber auch Katalysatoren wie Platin, Palladium, Palladium auf Tierkohle oder Raney-Nickel eingesetzt werden.

Der Katalysator wird in einer Menge von 0,00001 bis 1 mol, bevorzugt von 0,0001 bis 0,1 mol bezogen auf 1 mol der Verbindung der Formel (XIII) eingesetzt.

Die Verbindung der Formel (XIV) ist neu und kann nach der oben angegebenen Methode hergestellt werden.

Die Verbindung der Formel (XIII) ist bekannt (vgl. T. Watanabe et al., Chem. Pharm. Bull., 20(10), 2123-2127 (1972)).

Als Basen eignen sich beispielsweise Triethylamin, 1,8-Diazabicyclo[5.4.0]undec-7-en, Alkoholate wie Natriummethylat oder Carbonate wie Natriumcarbonat.

Die Base wird in katalytischen Mengen eingesetzt, vorzugsweise mit 0,001 mol bis 0,1 mol bezogen auf 1 mol der Verbindungen der allgemeinen Formel (XV).

Die Aldehyde der allgemeinen Formel (XV) sind bekannt oder können nach literaturbekannten Methoden hergestellt werden (vgl. M. Elliott et al., Pestic. Sci. 18(4), 223-228 (1987)).

Die Acetessigester der Formel (III) sind bekannt oder können nach üblichen Methoden hergestellt werden [vgl. D. Borrmann, "Umsetzung von Diketonen mit Alkoholen, Phenolen und Mercaptanen", in Houben-Weyl, Methoden der organischen Chemie, Vol. VIII/4, 230 ff. (1968)].

Die Ylidenverbindungen (IV) und (VII) sind neu, können aber nach üblichen Methoden hergestellt werden [vgl. H. Dornow und W. Sasssenberg, Liebigs Ann. Chem. 602, 14 (1957)].

Die Aminocrotonsäurederivate der Formel (VI) und (VIII) sind bekannt oder können nach bekannten Methoden hergestellt werden [S.A. Glickman, A.C. Cope, J. Am. Chem. Soc. 67, 1017 (1946)].

Die Verbindungen der allgemeinen Formel (XI) sind neu und können nach der oben aufgeführten Methode hergestellt werden.

Die Verbindungen der allgemeinen Formel (V) sind ebenfalls bekannt [vgl. N. Levy, C.W. Scaife, J. Chem. Soc. (London) 1946, 1100; C.D. Hurd, M.E. Nilson, J. Org. Chem. 20, 927 (1955)].

Die vorstehenden Herstellungsverfahren sind lediglich zur Verdeutlichung angegeben. Die Herstellung der Verbindungen der Formel (I) und (II) sind nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur. Sie können deshalb in Arzneimitteln zur Beeinflussung des pathologisch veränderten Blutdrucks, als Koronartherapeutika und zur Behandlung der Herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden.

Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. Als Perfusionsmedium dient eine Krebs-Henseleit-Lösung (1) (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2EDTA$), deren $CaCl_2$-Gehalt 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32° C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert (Opie, L., J. Physiol., 180 (1965), 529 - 541). Der Perfusionsdruck wird mittels eines Druckaufnehmers, der vor dem Herzen mit dem

Perfusionssystem in Verbindung steht, registriert. Unter diesen Bedingungen zeigt eine Senkung des Perfusionsdrucks eine Koronardilalation, eine zu- bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Substanzeffekte auf die Kontraktionsamplitude isolierter Meerschweinchen-Herzvorhöfe bei einer Wirkstoffkonzentration von $10^{-4}$ g/l.

| Bsp.-Nr. | %-Änderung der Ventrikeldruckamplitude |
|---|---|
| 3 | + 129 |
| 8 | + 129 |
| 29 | + 170 |
| 30 | + 79 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Beispiel I

4-Amino-3-hydroxyphthalid

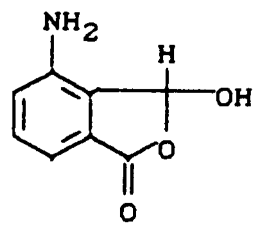

10 g 3-Hydroxy-4-nitro-phthalid werden in 100 ml Tetrahydrofuran gelöst und nach Zugabe von 1 g Palladium auf Bariumsulfat (5 %) bei Atmosphärendruck und 20-35° C hydriert. Es wird vom Katalysator abfiltriert und eingeengt. Der Eindampfrückstand wird mit Ether verrührt und abgesaugt. Man erhält 5,8 g (68,5 % der Theorie) einer farblosen Substanz vom Schmp. 280-285° C (Zers.).

Beispiel II

3-Phenyl-chinolin-5-carbonsäure

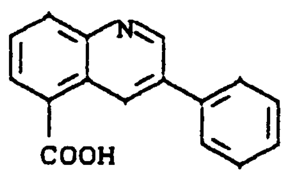

50 g (0,256 mol) 3-Hydroxy-4-nitro-phthalid werden in 380 ml Ethanol mit 5 g Pd/Bariumsulfat (5 %) bei 20° C bei 3 bar hydriert. Es wird abgesaugt, das Filtrat wird mit 0,308 mol = 38,7 ml Phenylacetaldehyd versetzt. Es wird 4 Stunden gekocht, wobei die Chinolincarbonsäure ausfällt. Es wird abgekühlt, abgesaugt und mit Ethanol gewaschen. Man erhält 28,3 g (44,3 % der Theorie) einer farblosen Verbindung vom Schmp. > 290° C.

Beispiel III

5-Hydroxymethyl-3-phenyl-chinolin

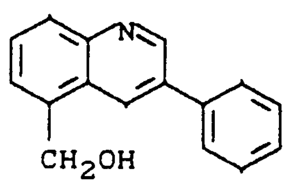

80 g (0,32 mol) 3-Phenyl-chinolin-5-carbonsäure werden unter Argon in 1,6 l abs. Tetrahydrofuran suspendiert und tropfenweise mit 400 ml 1 molarer Lithiumaluminiumhydrid-Lösung in Tetrahydrofuran versetzt. Dabei darf die Temperatur bis 35° C ansteigen. Es wird 90 Minuten nachgerührt und unter Kühlen tropfenweise mit 16 ml Wasser und danach mit 48 ml gesättigter Ammoniumchlorid-Lösung versetzt, 30 Minuten nachgerührt, abgesaugt, mit Tetrahydrofuran gewaschen und eingeengt. Man erhält ca. 80 g Rohprodukt. Der Schmelzpunkt der gereinigten Verbindung liegt bei 110-112° C.

Beispiel IV

2-Phenyl-chinolin-8-aldehyd

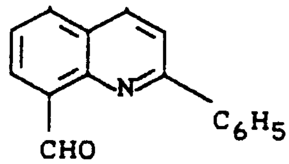

8,46 g 8-Methyl-2-phenylchinolin [vgl. Atwell, G.J. et al., J. Med. Chem. 32, 396-401 (1989)] werden mit 8,9 g (80 mmol) Selendioxid vermischt und unter Rühren zuerst auf 140° C, dann 5 Stunden auf 160° C erhitzt. Es wird abgekühlt, mit weiteren 2,5 g Selendioxid versetzt und 2,5 Stunden bei 160° C gerührt. Es wird abgekühlt, mit Methylenchlorid verrührt, abfiltriert und eingeengt. Das erhaltene Gemisch wird durch Flash-Chromatographie an Kieselgel mit Toluol/Hexan 1:1 und Toluol gereinigt. Man erhält 3,9 g beigefarbene Kristalle vom Schmelzpunkt 100-102° C.

Beispiel V

3-Phenyl-chinolin-5-aldehyd

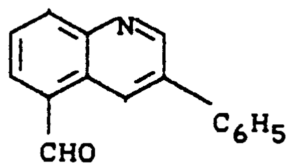

34,5 g (124,5 mmol) 3-Phenyl-chinolin-5-carbonsäureethylester [vgl. Makriyannis, A. et al., J. Med. Chem. 16, 118-122 (1973)] werden in 800 ml trockenem Methylenchlorid gelöst und auf ca. -70° C abgekühlt. Bei dieser Temperatur werden unter Argon 406 ml 1 molare DIBAL-Lösung in Toluol zugetropft. Es wird 2,5 Stunden bei -70° C nachgerührt und dann vorsichtig tropfenweise mit 40 ml Wasser versetzt. Es werden 200 ml gesättigte Kochsalzlösung zugetropft. Es wird langsam auf Raumtemperatur kommen gelassen und über Nacht gerührt. Es wird vom entstandenen Salz abgesaugt und mit Methylenchlorid gewaschen. Das Filtrat wird 1 mal mit Wasser geschüttelt, getrocknet und eingeengt. Man erhält 33 g eines Öls, welches in 600 ml Methylenchlorid gelöst wird und nach Zugabe von 150 g Mangandioxid 3 Stunden gekocht wird. Es wird abgekühlt und abgesaugt. Das Filtrat wird mit Tonsil versetzt, filtriert und wieder eingeengt. Man erhält 28,2 g beigefarbenes Produkt vom Schmelzpunkt 122-124° C.

Herstellungsbeispiele

Beispiel 1 (Herstellungsmethode B)

1,4-Dihydro-2,6-dimethyl-3-nitro-4-(3-phenyl-chinolin-5-yl)-pyridin-5-carbonsäuremethylester

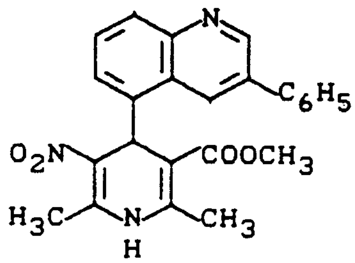

1,17 g (5 mmol) 3-Phenyl-chinolin-5-aldehyd werden in 10 ml Ethanol mit 0,9 g (8,75 mmol) Nitroaceton, 0,58 g (5 mmol) 3-Aminocrotonsäuremethylester und 0,3 ml (5 mmol) Essigsäure versetzt und 1 Stunde gekocht. Es wird abgekühlt und eingeengt. Der Eindampfrückstand wird über eine Kieselgelsäule mit Chloroform/Methanol-Gemischen gereinigt. Die sauberen Fraktionen werden eingeengt, der Eindampfrückstand wird aus Essigester umkristallisiert. Man erhält 0,6 g orangefarbener Kristalle vom Schmelzpunkt 262° C.

Beispiel 2 (Herstellungsmethode A)

1,4-Dihydro-2,6-dimethyl-4-(3-phenyl-chinolin-5-yl)-pyridin-3-carbonsäuremethylester-5-carbonsäurenitril

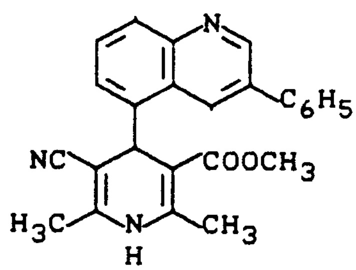

0,7 g (3 mmol) 3-Phenylchinolin-5-aldehyd werden in 6 ml Ethanol mit 0,35 g (3 mmol) Acetessigsäuremethylester und 0,25 g (3 mmol) 3-Aminocrotonsäurenitril 24 Stunden gekocht. Es wird abgekühlt und eingeengt. Der Eindampfrückstand wird über eine Kieselgelsäule mit Toluol/Essigester-Gemischen gereinigt. Die sauberen Fraktionen werden vereint und eingeengt. Durch Kristallisation aus Isopropanol werden 0,5 g farblose Substanz vom Schmelzpunkt 242° C erhalten.

Beispiel 3

2-Methyl-4-(3-phenyl-chinolin-5-yl)-5-oxo-1,4,5,7-tetrahydrofuro[3,4-b]-pyridin-3-carbonsäureethylester

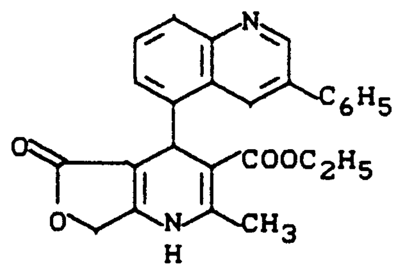

1,4 g (6,0 mmol) 3-Phenylchinolin-5-aldehyd werden in 20 ml Ethanol mit 0,79 ml (6,2 mmol) Acetessigsäureethylester und 1,16 g (6,2 mmol) 3-Amino-4-acetoxycrotonsäureethylester 2 Tage gekocht. Es wird abgekühlt und eingeengt. Es wird durch Flasch-Chromatographie grob gereinigt. Das erhaltene Zwischenprodukt wird in einer Lösung von 970 mg Kaliumhydroxid in 50 ml Methanol 40 Minuten gekocht. Es wird abgekühlt, mit 10%iger Salzsäure auf pH 5 gestellt, eingeengt, in Essigester aufgenommen und mit Wasser gewaschen. Es wird getrocknet und eingeengt. Es wird durch Säulenchromatographie von Nebenprodukten befreit, eingeengt und aus Acetonitril kristallisiert. Man erhält 460 mg farblose Kristalle vom Schmelzpunkt 257-260 ° C.

in Analogie zur Vorschrift der Beispiele 1, 2 und 3 wurden die in der Tabelle 1 aufgeführten Verbindungen hergestellt:

Tabelle 1:

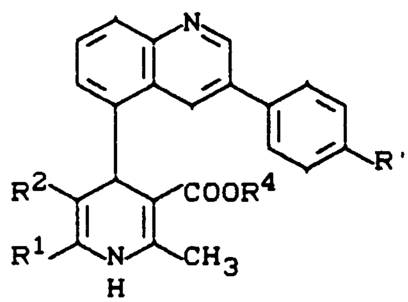

| Bsp.-Nr. | $R^1$ | $R^2$ | $R^4$ | R' | F (°C) |
|---|---|---|---|---|---|
| 4 | $CH_3$ | $NO_2$ | $-CH(CH_3)_2$ | H | 252 |
| 5 | $CH_3$ | CN | $-C_2H_5$ | H | 218 |
| 6 | $CH_3$ | CN | $-CH(CH_3)_2$ | H | 239 |
| 7 | $CH_3$ | $NO_2$ | $-C_2H_5$ | H | 263 |
| 8 | 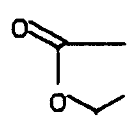 | | $-CH_3$ | H | 260 |

| Bsp.-Nr. | $R^1$ | $R^2$ | $R^4$ | R' | F (°C) |
|---|---|---|---|---|---|
| 9 | 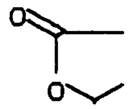 | | $-CH(CH_3)_2$ | H | 248 |
| 10 | $CH_3$ | CN | $-CH(CH_3)_2$ | H | (-)-Enant. 178° C |
| 11 | $CH_3$ | CN | $-CH(CH_3)_2$ | H | (+)-Enant. 178° C |
| 12 | $CH_3$ | CN | $-CH_2CH(CH_3)_2$ | H | 257-259 |
| 13 | $CH_3$ | CN | $-CH(CH_3)_2$ | F | (-)-Enant. 209° C |
| 14 | $CH_3$ | CN | $-CH(CH_3)_2$ | F | 213 |
| 15 | $CH_3$ | CN | $-CH(CH_3)-COOC_2H_5$ | H | 205-211 |
| 16 | $CH_3$ | CN | $-CH(CH_3)-CO_2-CH_2-CH(CH_3)_2$ | H | 157-158 |
| 17 | $CH_3$ | CN | $-(CH_2)_2-CH_3$ | H | 237 |
| 18 | $CH_3$ | CN | $-(CH_2)_2-O-CO-CH_3$ | H | 201-203 |
| 19 | $CH_3$ | CN | $-(CH_2)_2-OCH_3$ | H | 222 |
| 20 | $CH_3$ | CN | $-(CH_2)_2-CN$ | H | Zers. 193 |
| 21 | $CH_3$ | CN | $-(CH_2)_2-O-C_2H_5$ | F | 216-218 |
| 22 | $CH_3$ | CN | $-CH_3$ | H | (-)-Enant. 264° C |
| 23 | $CH_3$ | CN | $-C_2H_5$ | H | (-)-Enant. 213° C |
| 24 | $CH_3$ | CN | H | H | 244-246 |
| 25 | $CH_3$ | CN | $-(CH_2)_2-OH$ | H | 225-226 |

Beispiel 26

1,4-Dihydro-2,6-dimethyl-3-nitro-4-(2-phenylchinolin-8-yl)-pyridin-5-carbonsäureisopropylester

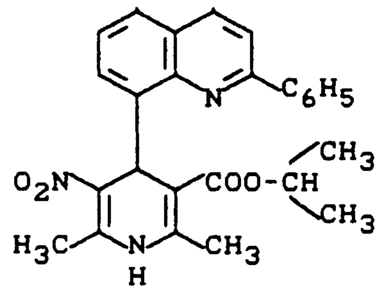

1,17 g (5 mmol) 2-Phenyl-chinolin-8-aldehyd werden in 10 ml Ethanol mit 0,9 g (8,75 mmol) Nitroaceton, 0,72 g (5 mmol) 3-Aminocrotonsäureisopropylester und 0,3 ml (5 mmol) Essigsäure 1 Stunde gekocht. Die ausgefallenen Kristalle werden nach dem Abkühlen abgesaugt und mit Ethanol gewaschen. Man erhält 1,1 g orangefarbener Kristalle vom Schmelzpunkt 205° C.

In Analogie zu der Vorschrift für Beispiel 26 wurden die in Tabelle 2 aufgeführten Beispiele hergestellt:

Tabelle 2

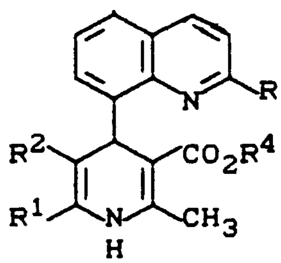

| Bsp.-Nr. | $R^1$ | $R^2$ | $R^4$ | R | F (°C) |
|---|---|---|---|---|---|
| 27 | 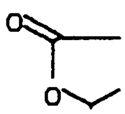 | | $-C_2H_5$ | 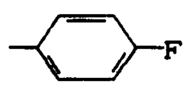 | 275-276 |
| 28 | $CH_3$ | $NO_2$ | $-C_2H_5$ | 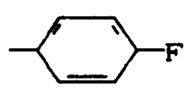 | 275-277 |
| 29 | $CH_3$ | $NO_2$ | $-CH_3$ | 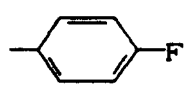 | 237 |
| 30 | 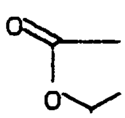 | | $-C_2H_5$ | 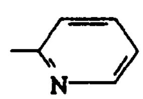 | 281 |

In Analogie zur Vorschrift des Beispiels 2 wurden die in den Tabellen 3, 4 und 5 aufgeführten Beispiele hergestellt.

**Tabelle 3:**

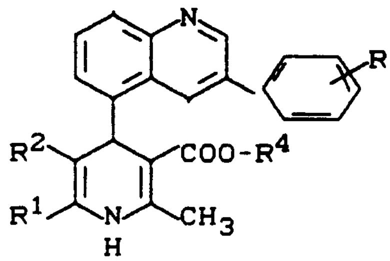

| Bsp.-Nr. | $R^1$ | $R^2$ | $R^4$ | R | Schmp. (°C) | $R_f$ (Toluol→Essigester 1:1) |
|---|---|---|---|---|---|---|
| 31 | $CH_3$ | CN | 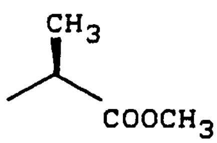 | H | - | 0,22/0,29 |
| 32 | $CH_3$ | CN | 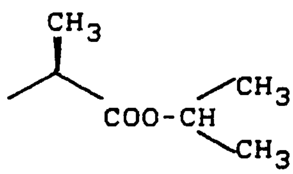 | H | 237-40 (+)-Enant. | 0,31 |
| 33 | $CH_3$ | CN | $-(CH_2)_2-O-C_2H_5$ | H | 232 | |
| 34 | $CH_3$ | CN | 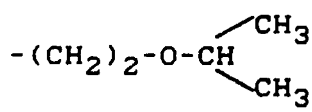 | H | 236 | |
| 35 | $CH_3$ | CN | 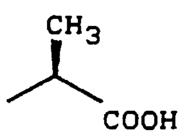 | H | 186-88 (-)-Enant. | |

Tabelle 3 (Fortsetzung)

| Bsp.-Nr. | $R^1$ | $R^2$ | $R^4$ | R | Schmp. (°C) | $R_f$ (Toluol→Essigester 1:1) |
|---|---|---|---|---|---|---|
| 36 | $CH_3$ | CN | 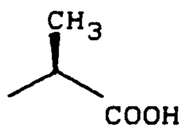 | H | 180 Zers. (+)-Enant. | |
| 37 | $CH_3$ | CN | 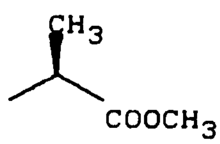 | H | 162 (-)-Enant. | 0,29 |
| 38 | $CH_3$ | CN | 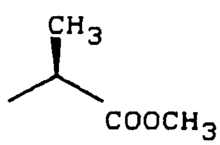 | H | (+)-Enant. | 0,22 |
| 39 | $CH_3$ | CN | 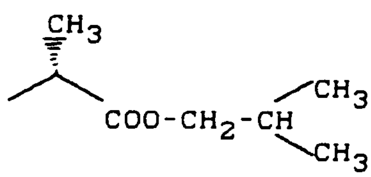 | H | - | 0,31 |
| 40 | $CH_3$ | CN | $-CH_2-CH_2-CH_3$ | H | 121 (-)-Enant. | |
| 41 | $CH_3$ | CN | $-CH_2-CH_2-CH_2-CH_3$ | H | 225 (-)-Enant. | |
| 42 | $CH_3$ | CN | 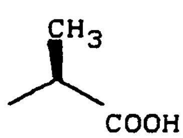 | 4-F | 182 (-)-Enant. | |
| 43 | $CH_3$ | CN | $-CH_2-CH_2-CH_3$ | 4-F | 115 (-)-Enant. | |

Tabelle 3 (Fortsetzung)

| Bsp.-Nr. | $R^1$ | $R^2$ | $R^4$ | R | Schmp. (°C) | $R_f$ (Toluol→Essigester 1:1) |
|---|---|---|---|---|---|---|
| 44 | $CH_3$ | CN | $-CH_3$ | 4-F | 245 (-)-Enant. | |
| 45 | $CH_3$ | CN | $-CH_2-CH_3$ | 4-F | 143 (-)-Enant. | |
| 46 | $CH_3$ | CN | $-CH_2-CH_2-CH_2-CH_3$ | 4-F | 204 (-)-Enant. | |
| 47 | $CH_3$ | CN | $-CH_2-CH_3$ | 4-$CH_3$ | 235 | |
| 48 | $CH_3$ | CN | 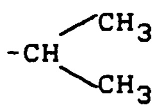 | 4-$CH_3$ | 242 | |
| 49 | $CH_3$ | CN | $-CH_2-CH_2-CH_3$ | 4-$CH_3$ | 236 | |
| 50 | $CH_3$ | CN | $-CH_3$ | 2-Cl | 249 | |
| 51 | $CH_3$ | CN | 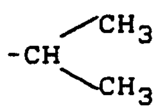 | 2-Cl | 179-81 | |
| 52 | $CH_3$ | CN | $-CH_2-CH_3$ | 2-Cl | 213 | |
| 53 | $CH_3$ | CN | $-CH_2-CH_2-CH_3$ | 2-Cl | 139 | |
| 54 | $CH_3$ | CN | $-CH_3$ | 2-F | 144-46 | |
| 55 | $CH_3$ | CN | $-CH_2-CH_3$ | 2-F | 181-83 | |
| 56 | $CH_3$ | CN | 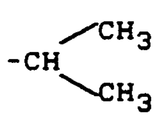 | 2-F | 192-94 | |

Tabelle 3 (Fortsetzung)

| Bsp.-Nr. | $R^1$ | $R^2$ | $R^4$ | R | Schmp. (°C) | $R_f$ (Toluol→Essigester 1:1) |
|---|---|---|---|---|---|---|
| 57 | $CH_3$ | CN | $-(CH_2)_2OCH_3$ | H | 173 (-)-Enant. | |
| 58 | $CH_3$ | CN | $-CH(CH_3)_2$ | m-F | 249 | |
| 59 | $CH_3$ | CN | $-CH_2-CH_2(CH_3)_2$ | H | 248 (-)-Enant. | |
| 60 | $CH_3$ | CN | $-C_2H_5$ | m-F | 210-212 | |
| 61 | $CH_3$ | CN | $-(CH_2)_2-CH_3$ | m-Cl | 214 | |
| 62 | $CH_3$ | CN | $-(CH_2)_2-OC_2H_5$ | H | 168 (-)-Enant. | |
| 63 | $CH_3$ | CN | $-CH(CH_3)_2$ | m-Cl | 270 | |
| 64 | $CH_3$ | CN | $-CH_3$ | m-Cl | ab 128 (Zers) | |
| 65 | $CH_3$ | CN | $-C_2H_5$ | m-Cl | 245 | |

Tabelle 4

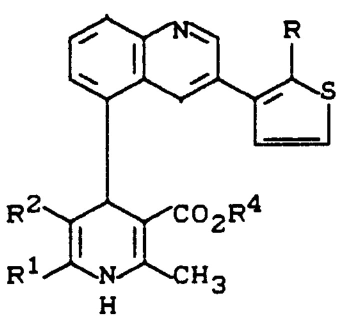

Tabelle 4 (Fortsetzung)

| Bsp.-Nr. | $R^1$ | $R^2$ | $R^4$ | R | Schmp. (°C) |
|---|---|---|---|---|---|
| 66 | $CH_3$ | $NO_2$ | $-C_2N_5$ | H | 246 (Zers.) |
| 67 | $CH_3$ | $NO_2$ | $-CH(CH_3)_2$ | Cl | 239 |

Tabelle 5

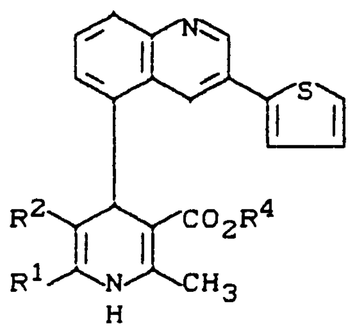

| Bsp. Nr. | $R^1$ | $R^2$ | $R^4$ | m.p. (°C) |
|---|---|---|---|---|
| 68 | $CH_3$ | $NO_2$ | $-CH(CH_3)_2)$ | 265 (Zers.) |

**Patentansprüche**

**Patentansprüche für folgende Vertragsstaaten : AT, BE, CH, DE, DK, FR, GB, GR, IT, LI, LU, NL, SE**

**1.** 4-Chinolyl-dihydropyridine der allgemeinen Formel (I)

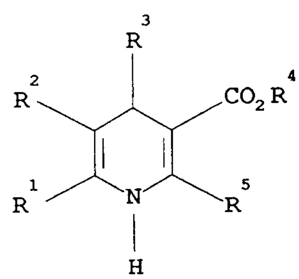

(I)

in welcher

$R^1$ und $R^5$ gleich oder verschieden sind und für geradkettiges oder verzweigtes Alkyl mit bis zu 6 Kohlenstoffatomen stehen,

$R^2$ für Nitro oder Cyano steht, oder

$R^1$ und $R^2$ gemeinsam einen Lactonring der Formel

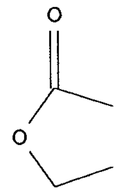

bilden,

$R^3$ für einen Rest der Formel

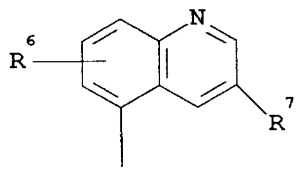

oder

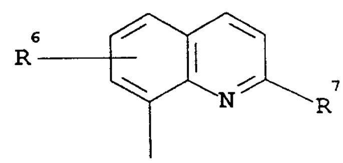

steht,

worin

$R^6$ Wasserstoff, Fluor, Chlor oder geradkettiges oder verzweigtes Alkyl oder Alkoxy mit jeweils bis zu 2 Kohlenstoffatomen bedeutet,

$R^7$ Phenyl bedeutet, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen substituiert ist, oder Thienyl oder Pyridyl bedeutet, die gegebenenfalls durch Fluor oder Chlor monosubstituiert sind,

$R^4$ für Wasserstoff oder

für geradkettiges oder verzweigtes Alkyl oder Alkenyl mit jeweils bis zu 8 Kohlenstoffatomen steht, die gegebenenfalls durch Fluor, Chlor, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 6 Kohlenstoffatomen oder durch Phenoxy oder Phenyl substituiert sind

und deren physiologisch unbedenklichen Salze.

**2.** Verbindungen der allgemeinen Formel I gemäß Anspruch 1, in welcher

$R^1$, $R^2$, $R^1$ und $R^2$ gemeinsam, $R^5$ sowie $R^3$ die in Anspruch 1 angegebene Bedeutung haben wobei

$R^6$ Wasserstoff, Chlor oder Methyl bedeutet,

$R^7$

- Phenyl bedeutet, das gegebenenfalls durch Fluor, Chlor, Nitro, Trifluormethyl oder durch geradkettiges oder verzweigtes Alkyl oder Alkoxy mit jeweils bis zu 4 Kohlenstoffatomen substituiert ist, oder
- Pyridyl oder Thienyl bedeutet, die gegebenenfalls durch Fluor oder Chlor monosubstituiert wird,

$R^4$

- für Wasserstoff steht, oder
- für geradkettiges oder verzweigtes Alkyl mit bis zu 6 Kohlenstoffatomen steht, das gegebenenfalls durch Hydroxy, Carboxy, Cyano oder durch geradkettiges oder verzweigtes Alkoxycarbonyl, Alkoxy oder Acyloxy mit jeweils bis zu 4 Kohlenstoffatomen substituiert ist

**3.** Verfahren zur Herstellung von Verbindungen der allgemeinen Formel I gemäß Anspruch 1, dadurch gekennzeichnet, daß man im Fall, daß $R^1$ und $R^2$ die oben angegebene Bedeutung haben, aber nicht gemeinsam einen Lactonring bilden,

[A] Verbindungen der allgemeinen Formel (II)

$R^3$-CHO (II)

in welcher
$R^3$ die oben angegebene Bedeutung hat,
zunächst mit Acetessigestern der allgemeinen Formel (III)

$R^5$-CO-$CH_2$-$CO_2$-$R^4$ (III)

in welcher
$R^4$ und $R^5$ die oben angegebene Bedeutung haben,
gegebenenfalls unter Isolierung der entsprechenden Ylidenverbindungen der allgemeinen Formel (IV)

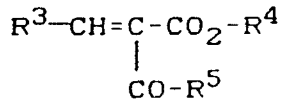 (IV)

in welcher
$R^3$, $R^4$ und $R^5$ die oben angegebene Bedeutung haben,
umsetzt und anschließend
entweder mit Verbindungen der Formel (V)

$R^1$-CO-$CH_2$-$R^2$ (V)

$R^1$ und $R^2$ die oben angegebene Bedeutung haben,
in Anwesenheit von Ammoniak oder Ammoniumsalzen,
oder direkt mit Aminoderivaten der allgemeinen Formel (VI)

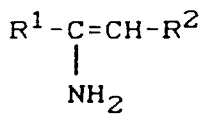 (VI)

in welcher
$R^1$ und $R^2$ die oben angegebene Bedeutung haben,
gegebenenfalls in Gegenwart von inerten organischen Losemitteln umsetzt,
oder
[B] die Aldehyde der allgemeinen Formel (II) zunächst mit den Verbindungen der allgemeinen Formel (V),
gegebenenfalls unter Isolierung der Ylidenverbindungen der allgemeinen Formel (VII)

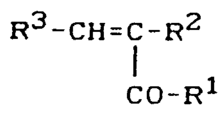 (VII)

in welcher
$R^1$, $R^2$ und $R^3$ die oben angegebene Bedeutung haben,
umsetzt und in einem nächsten Schritt mit den oben aufgeführten Verbindungen der allgemeinen Formel (III) in inerten Lösemitteln, in Anwesenheit von Ammoniak oder Ammoniumsalzen oder direkt mit Enaminocarbonsäurederivaten der allgemeinen Formel (VIII)

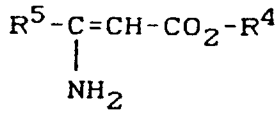

(VIII)

in welcher

$R^4$ und $R^5$ die oben angegebene Bedeutung haben,

umsetzt,

oder im Fall, daß $R^1$ und $R^2$ gemeinsam einen Lactonring bilden,

[C] zunächst nach den unter [A] und [B] aufgeführten Methoden, Verbindungen der allgemeinen Formel (Ia)

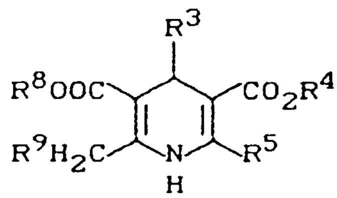

(Ia)

in welcher

$R^3$, $R^4$ und $R^5$ die oben angegebene Bedeutung haben,

$R^8$ für einen $C_1$-$C_6$-Alkyl-Rest steht

und

$R^9$ für eine übliche Abgangsgruppe steht,

herstellt und nach bekannten Verfahren einen säure-oder basenkatalysierten Ringschluß anschließt, und im Fall, daß $R^4$ nicht Wasserstoff bedeutet,

[D] Verbindungen der allgemeinen Formel (I), in welcher $R^1$, $R^2$, $R^3$, $R^4$ und $R^5$ die oben angegebene Bedeutung haben und $R^4$ für Wasserstoff steht, gegebenenfalls über ein reaktives Säurederivat, mit den entsprechenden Alkoholen umsetzt, wobei durch Einsatz der enantiomerenreinen Carbonsäuren ($R^4$ = H) die entsprechenden Enantiomere der Ester erhalten werden.

**4.** Aldehyde der allgemeinen Formel II

$R^3$—CHO (II)

in welcher

$R^3$ für einen Rest der Formel

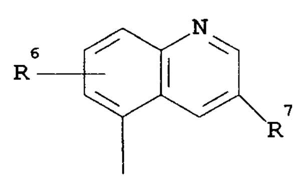

steht,

worin $R^6$ und $R^7$ wie im Anspruch 1 definiert sind.

**5.** Verfahren zur Herstellung von Aldehyden der allgemeinen Formel II gemäß Anspruch 4, dadurch gekennzeichnet, daß man

a) entweder Verbindungen der allgemeinen Formel (IX)

$R^3$—$CH_3$ (III)

in welcher $R^3$ die oben angegebene Bedeutung hat,

direkt zu Verbindungen der Formel (II) oxidiert, oder zuerst zu Verbindungen der allgemeinen Formel

(X)

$R^3—CH_2—Hal$ (X)

in welcher $R^3$ die oben angegebene Bedeutung hat, und
Hal für Halogen, steht,
halogeniert, anschließend mit Acetationen umsetzt und zu Verbindungen der Formel (XI)

$R^3-CH_2-OH$ (XI)

in welcher
$R^3$ die oben angegebene Bedeutung hat,
verseift und diese dann zu Verbindungen der Formel (II) oxidiert,
oder daß man
b) Verbindungen der allgemeinen Formel (XII),

$R^3-COOR$ (XII)

in welcher
$R^3$ die oben angegebene Bedeutung hat
und
R
- für Wasserstoff oder einen $C_1-C_6$-Alkylrest steht,
entweder direkt reduziert oder durch Reduktion zunächst Verbindungen der Formel (XI) herstellt und diese dann oxidiert.

**6.** Arzneimittel enthaltend mindestens eine Verbindung der allgemeinen Formel I gemäß Anspruch 1.

**7.** Verfahren zur Herstellung von Arzneimitteln, dadurch gekennzeichnet, daß man mindestens eine Verbindung der allgemeinen Formel I gemäß Anspruch 1, gegebenenfalls unter Verwendung von üblichen Hilfs- und Trägerstoffen in eine geeignete Applikationsform überführt.

**8.** Verwendung von Verbindungen der allgemeinen Formel I gemäß Anspruch 1, bei der Herstellung von Arzneimitteln.

**Patentansprüche für folgenden Vertragsstaat : ES**

**1.** Verfahren zur Herstellung von 4-Chinolyldihydropyridinen der allgemeinen Formel I

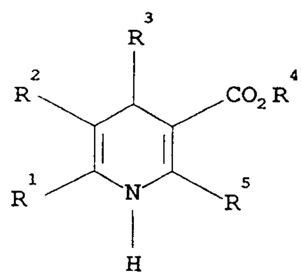

(I)

in welcher
$R^1$ und $R^5$ gleich oder verschieden sind und für geradkettiges oder verzweigtes Alkyl mit bis zu 6 Kohlenstoffatomen stehen,
$R^2$ für Nitro oder Cyano steht, oder
$R^1$ und $R^2$ gemeinsam einen Lactonring der Formel

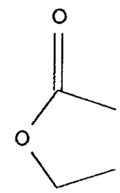

bilden,

$R^3$ für einen Rest der Formel

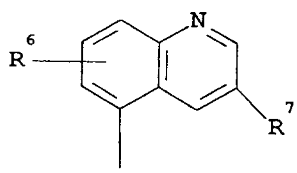 oder 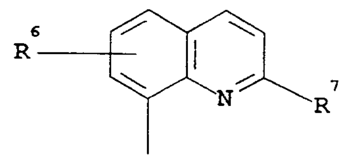

steht,
worin

$R^6$ Wasserstoff, Fluor, Chlor oder geradkettiges oder verzweigtes Alkyl oder Alkoxy mit jeweils bis zu 2 Kohlenstoffatomen bedeutet,

$R^7$ Phenyl bedeutet, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen substituiert ist, oder Thienyl oder Pyridyl bedeutet, die gegebenenfalls durch Fluor oder Chlor monosubstituiert sind,

$R^4$ für Wasserstoff oder
für geradkettiges oder verzweigtes Alkyl oder Alkenyl mit jeweils bis zu 8 Kohlenstoffatomen steht, die gegebenenfalls durch Fluor, Chlor, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 6 Kohlenstoffatomen oder durch Phenoxy oder Phenyl substituiert sind

und deren physiologisch unbedenklichen Salze, dadurch gekennzeichnet, daß man im Fall, daß $R^1$ und $R^2$ die oben angegebene Bedeutung haben, aber nicht gemeinsam einen Lactonring bilden,

[A] Verbindungen der allgemeinen Formel (II)

$R^3$-CHO (II)

in welcher
$R^3$ die oben angegebene Bedeutung hat,
zunächst mit Acetessigestern der allgemeinen Formel (III)

$R^5$-CO-$CH_2$-$CO_2$-$R^4$ (III)

in welcher
$R^4$ und $R^5$ die oben angegebene Bedeutung haben,
gegebenenfalls unter Isolierung der entsprechenden Ylidenverbindungen der allgemeinen Formel (IV)

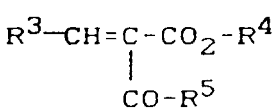 (IV)

in welcher
$R^3$, $R^4$ und $R^5$ die oben angegebene Bedeutung haben,

umsetzt und anschließend
entweder mit Verbindungen der Formel (V)

$R^1$-CO-$CH_2$-$R^2$ (V)

$R^1$ und $R^2$ die oben angegebene Bedeutung haben,
in Anwesenheit von Ammoniak oder Ammoniumsalzen,
oder direkt mit Aminoderivaten der allgemeinen Formel (VI)

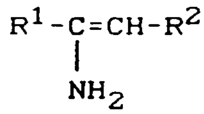
(VI)

in welcher
$R^1$ und $R^2$ die oben angegebene Bedeutung haben,
gegebenenfalls in Gegenwart von inerten organischen Lösemitteln umsetzt,
oder
[B] die Aldehyde der allgemeinen Formel (II) zunächst mit den Verbindungen der allgemeinen Formel (V),
gegebenenfalls unter Isolierung der Ylidenverbindungen der allgemeinen Formel (VII)

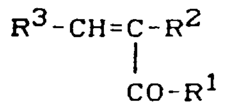
(VII)

in welcher
$R^1$, $R^2$ und $R^3$ die oben angegebene Bedeutung haben,
umsetzt und in einem nächsten Schritt mit den oben aufgeführten Verbindungen der allgemeinen Formel (III) in inerten Lösemitteln, in Anwesenheit von Ammoniak oder Ammoniumsalzen oder direkt mit Enaminocarbonsäurederivaten der allgemeinen Formel (VIII)

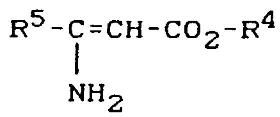
(VIII)

in welcher
$R^4$ und $R^5$ die oben angegebene Bedeutung haben,
umsetzt,
oder im Fall, daß $R^1$ und $R^2$ gemeinsam einen Lactonring bilden,
[C] zunächst nach denen unter [A] und [B] aufgeführten Methoden, Verbindungen der allgemeinen Formel (Ia)

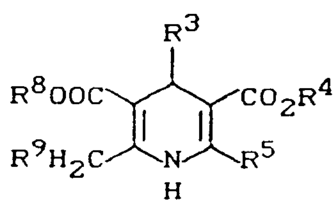
(Ia)

in welcher
$R^3$, $R^4$ und $R^5$ die oben angegebene Bedeutung haben,

$R^8$ für einen $C_1$-$C_6$-Alkyl-Rest steht
und
$R^9$ für eine übliche Abgangsgruppe steht,
herstellt und nach bekannten Verfahren einen säure-oder basenkatalysierten Ringschluß anschließt, und im Fall, daß $R^4$ nicht Wasserstoff bedeutet,
[D] Verbindungen der allgemeinen Formel (I), in welcher $R^1$, $R^2$, $R^3$, $R^4$ und $R^5$ die oben angegebene Bedeutung haben und $R^4$ für Wasserstoff steht, gegebenenfalls über ein reaktives Säurederivat, mit den entsprechenden Alkoholen umsetzt, wobei durch Einsatz der enantiomerenreinen Carbonsäuren ($R^4$ = H) die entsprechenden Enantiomere der Ester erhalten werden.

**2.** Verfahren zur Herstellung von Aldehyden der allgemeinen Formel II

$R^3$—CHO (II)

in welcher
$R^3$ für einen Rest der Formel

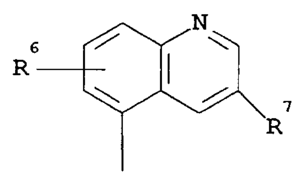

steht,
worin $R^6$ und $R^7$ wie in Anspruch 1 definiert sind,
dadurch gekennzeichnet, daß man
a) entweder Verbindungen der allgemeinen Formel (IX)

$R^3$—$CH_3$ (III)

in welcher $R^3$ die oben angegebene Bedeutung hat,
direkt zu Verbindungen der Formel (II) oxidiert, oder zuerst zu Verbindungen der allgemeinen Formel (X)

$R^3$—$CH_2$— Hal (X)

in welcher $R^3$ die oben angegebene Bedeutung hat, und
Hal für Halogen, steht,
halogeniert, anschließend mit Acetationen umsetzt und zu Verbindungen der Formel (XI)

$R^3$-$CH_2$-OH (XI)

in welcher
$R^3$ die oben angegebene Bedeutung hat,
verseift und diese dann zu Verbindungen der Formel (II) oxidiert,
oder daß man
b) Verbindungen der allgemeinen Formel (XII),

$R^3$-COOR (XII)

in welcher
$R^3$ die oben angegebene Bedeutung hat
und
R
- für Wasserstoff oder einen $C_1$-$C_6$-Alkylrest steht,
entweder direkt reduziert oder durch Reduktion zunächst Verbindungen der Formel (XI) herstellt und diese dann oxidiert.

3. Verfahren zur Herstellung von Arzneimitteln, dadurch gekennzeichnet, daß man mindestens eine Verbindung der allgemeinen Formel I gemäß Anspruch 1, gegebenenfalls unter Verwendung von üblichen Hilfs- und Trägerstoffen in eine geeignete Applikationsform überführt.

## Claims

**Claims for the following Contracting States : AT, BE, CH, DE, DK, FR, GB, GR, IT, LI, LU, NL, SE**

1. 4-Quinolyl-dihydropyridines of the general formula (I)

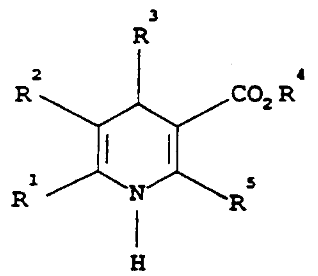

(I)

(I)

in which

$R^1$ and $R^5$ are identical or different and represent straight-chain or branched alkyl having up to 6 carbon atoms,

$R^2$ represents nitro or cyano, or

$R^1$ and $R^2$ together form a lactone ring of the formula

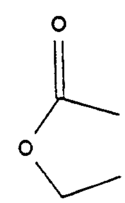

$R^3$ represents a radical of the formula

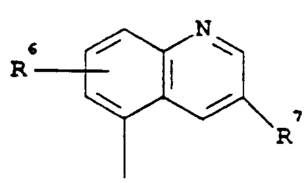

or

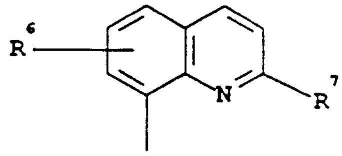

in which

$R^6$ denotes hydrogen, fluorine, chlorine or straight-chain or branched alkyl or alkoxy in each case having up to 2 carbon atoms,

$R^7$ denotes phenyl which is optionally substituted by fluorine, chlorine, nitro, cyano, trifluoromethyl or by straight-chain or branched alkyl or alkoxy in each case having up to 6 carbon atoms, or denotes thienyl or pyridyl which are optionally monosubstituted by fluorine or chlorine,

$R^4$ represents hydrogen or represents straight-chain or branched alkyl or alkenyl in each case having up to 8 carbon atoms, which are optionally substituted by fluorine, chlorine, hydroxyl, carboxyl, cyano, nitro or by straight-chain or branched alkylthio, alkoxy, alkoxycarbonyl, acyl or acyloxy in each case having up to 6 carbon atoms or by phenoxy or phenyl

and their physiologically acceptable salts.

**2.** Compounds of the general formula I according to Claim 1, in which $R^1$, $R^2$, $R^1$ and $R^2$ together, $R^5$ and $R^3$ have the meaning given in Claim 1, where

$R^6$ denotes hydrogen, chlorine or methyl,

$R^7$
- denotes phenyl which is optionally substituted by fluorine, chlorine, nitro, trifluoromethyl or by straight-chain or branched alkyl or alkoxy in each case having up to 4 carbon atoms, or
- denotes pyridyl or thienyl, which are optionally monosubstituted by fluorine or chlorine,

$R^4$
- represents hydrogen, or
- represents a straight-chain or branched alkyl having up to 6 carbon atoms, which is optionally substituted by hydroxyl, carboxyl, cyano or by straight-chain or branched alkoxycarbonyl, alkoxy or acyloxy in each case having up to 4 carbon atoms.

**3.** Process for the preparation of compounds of the general formula I according to Claim 1, characterized in that in the case in which $R^1$ and $R^2$ have the abovementioned meaning, but do not together form a lactone ring,

[A] compounds of the general formula (II)

$R^3$-CHO (II)

in which

$R^3$ has the abovementioned meaning,

are first reacted with acetoacetates of the general formula (III)

$R^5$-CO-$CH_2$-$CO_2$-$R^4$ (III)

in which

$R^4$ and $R^5$ have the abovementioned meaning,

if appropriate with the isolation of the corresponding ylidene compounds of the general formula (IV)

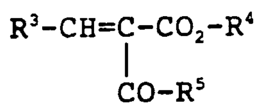

(IV)

in which

$R^3$, $R^4$ and $R^5$ have the abovementioned meaning,

and are then reacted

either with compounds of the formula (V)

$R^1$-CO-$CH_2$-$R^2$ (V)

in which

$R^1$ and $R^2$ have the abovementioned meaning,

in the presence of ammonia or ammonium salts, or directly with amino derivatives of the general formula (VI)

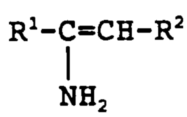

(VI)

in which

$R^1$ and $R^2$ have the abovementioned meaning,

if appropriate in the presence of inert organic solvents,

or

[B] the aldehydes of the general formula (II) are first reacted with the compounds of the general formula (V),
if appropriate with the isolation of the ylidene compounds of the general formula (VII)

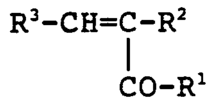

(VII)

in which
$R^1$, $R^2$ and $R^3$ have the abovementioned meaning,
and in a next step are reacted with the above-mentioned compounds of the general formula (III) in inert solvents, in the presence of ammonia or ammonium salts or directly with enaminocarboxylic acid derivatives of the general formula (VIII)

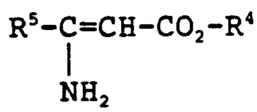

(VIII)

in which
$R^4$ and $R^5$ have the abovementioned meaning,
or in the case in which $R^1$ and $R^2$ together form a lactone ring,
[C] first, according to the methods mentioned under [A] and [B], compounds of the general formula (Ia)

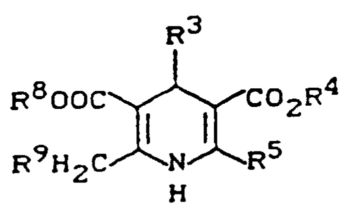

(Ia)

in which
$R^3$, $R^4$ and $R^5$ have the abovementioned meaning,
$R^8$ represents a $C_1$-$C_6$-alkyl radical
and
$R^9$ represents a customary leaving group,
are prepared and an acid- or base-catalysed ring closure according to known processes is added,
and in the case in which $R^4$ does not denote hydrogen,
[D] compounds of the general formula (I), in which $R^1$, $R^2$, $R^3$, $R^4$ and $R^5$ have the abovementioned meaning and $R^4$ represents hydrogen, are reacted, if appropriate via a reactive acid derivative, with the corresponding alcohols, the corresponding enantiomers of the esters being obtained by use of the enantiomerically pure carboxylic acids ($R^4$ = H).

**4.** Aldehydes of the general formula II

$R^3$—CHO (II)

in which
$R^3$ represents a radical of the formula

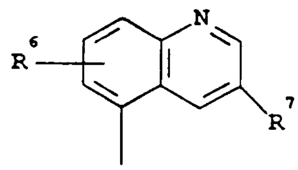

in which $R^6$ and $R^7$ are defined as in Claim 1.

**5.** Process for the preparation of aldehydes of the general formula II according to Claim 4, characterized in that

a) either compounds of the general formula (IX)

$R^3—CH_3$ (IX)

in which $R^3$ has the abovementioned meaning, are oxidized directly to compounds of the formula (II), or first halogenated to give compounds of the general formula (X)

$R^3—CH_2—Hal$ (X)

in which $R^3$ has the abovementioned meaning, and

Hal represents halogen,

then reacted with acetate ions and hydrolysed to give compounds of the formula (XI)

$R^3$-$CH_2$-OH (XI)

in which

$R^3$ has the abovementioned meaning,

and these are then oxidized to compounds of the formula (II),

or in that

b) compounds of the general formula (XII)

$R^3$-COOR (XII)

in which

$R^3$ has the abovementioned meaning

and

R - represents hydrogen or a $C_1$-$C_6$-alkyl radical,

are either directly reduced or compounds of the formula (XI) are first prepared by reduction and these are then oxidized.

**6.** Medicaments containing at least one compound of the general formula I according to Claim 1.

**7.** Process for the production of medicaments, characterized in that at least one compound of the general formula I according to Claim 1 is converted into a suitable form for administration, optionally using customary auxiliaries and excipients.

**8.** Use of compounds of the general formula I according to Claim 1 in the production of medicaments.

**Claims for the following Contracting State : ES**

**1.** Process for the preparation of 4-quinolyldihydropyridines of the general formula I

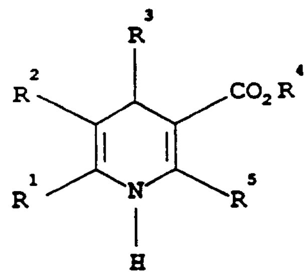

(I)

in which

$R^1$ and $R^5$ are identical or different and represent straight-chain or branched alkyl having up to 6 carbon atoms,

$R^2$ represents nitro or cyano, or

$R^1$ and $R^2$ together form a lactone ring of the formula

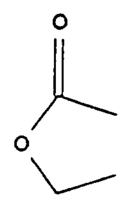

$R^3$ represents a radical of the formula

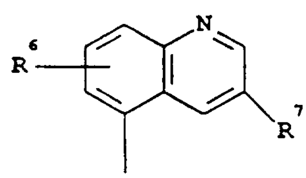

or

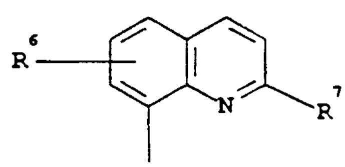

in which

$R^6$ denotes hydrogen, fluorine, chlorine or straight-chain or branched alkyl or alkoxy in each case having up to 2 carbon atoms,

$R^7$ denotes phenyl which is optionally substituted by fluorine, chlorine, nitro, cyano, trifluoromethyl or by straight-chain or branched alkyl or alkoxy in each case having up to 6 carbon atoms, or denotes thienyl or pyridyl, which are optionally monosubstituted by fluorine or chlorine,

$R^4$ represents hydrogen or represents straight-chain or branched alkyl or alkenyl in each case having up to 8 carbon atoms, which are optionally substituted by fluorine, chlorine, hydroxyl, carboxyl, cyano, nitro or by straight-chain or branched alkylthio, alkoxy, alkoxycarbonyl, acyl or acyloxy in each case having up to 6 carbon atoms or by phenoxy or phenyl

and their physiologically acceptable salts, characterized in that in the case in which $R^1$ and $R^2$ have the above-mentioned meaning, but do not together form a lactone ring,

[A] compounds of the general formula (II)

$R^3$-CHO (II)

in which
$R^3$ has the abovementioned meaning,
are first reacted with acetoacetates of the general formula (III)

$R^5$-CO-$CH_2$-$CO_2$-$R^4$ (III)

in which
$R^4$ and $R^5$ have the abovementioned meaning,
if appropriate with the isolation of the corresponding ylidene compounds of the general formula (IV)

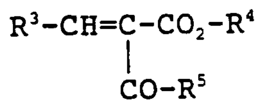
(IV)

in which
$R^3$, $R^4$ and $R^5$ have the abovementioned meaning,
and are then reacted
either with compounds of the formula (V)

$R^1$-CO-$CH_2$-$R^2$ (V)

in which
$R^1$ and $R^2$ have the abovementioned meaning,
in the presence of ammonia or ammonium salts, or directly with amino derivatives of the general formula (VI)

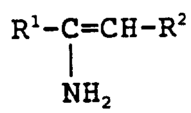
(VI)

in which
$R^1$ and $R^2$ have the abovementioned meaning,
if appropriate in the presence of inert organic solvents,

or

[B] the aldehydes of the general formula (II) are first reacted with the compounds of the general formula (V),
if appropriate with the isolation of the ylidene compounds of the general formula (VII)

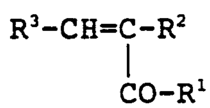
(VII)

in which
$R^1$, $R^2$ and $R^3$ have the abovementioned meaning,
and in a next step are reacted with the above-mentioned compounds of the general formula (III) in inert solvents, in the presence of ammonia or ammonium salts or directly with enaminocarboxylic acid derivatives of the general formula (VIII)

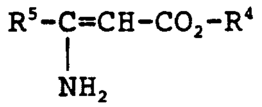 (VIII)

in which

$R^4$ and $R^5$ have the abovementioned meaning,

or in the case in which $R^1$ and $R^2$ together form a lactone ring,

[C] first, according to the methods mentioned under [A] and [B], compounds of the general formula (Ia)

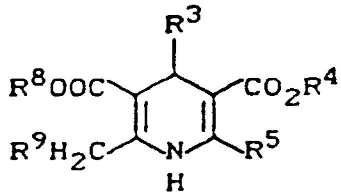 (Ia)

in which

$R^3$, $R^4$ and $R^5$ have the abovementioned meaning,

$R^8$ represents a $C_1$-$C_6$-alkyl radical

and

$R^9$ represents a customary leaving group,

are prepared and an acid- or base-catalysed ring closure according to known processes is added,

and in the case in which $R^4$ does not denote hydrogen,

[D] compounds of the general formula (I), in which $R^1$, $R^2$, $R^3$, $R^4$ and $R^5$ have the abovementioned meaning and $R^4$ represents hydrogen, are reacted, if appropriate via a reactive acid derivative, with the corresponding alcohols, the corresponding enantiomers of the esters being obtained by use of the enantiomerically pure carboxylic acids ($R^4$ = H).

**2.** Process for the preparation of aldehydes of the general formula II

$R^3$—CHO (II)

in which

$R^3$ represents a radical of the formula

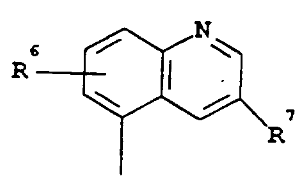

in which $R^6$ and $R^7$ are defined as in Claim 1,

characterized in that

a) either compounds of the general formula (IX)

$R^3$—$CH_3$ (IX)

in which $R^3$ has the abovementioned meaning,

are oxidized directly to compounds of the formula (II), or first halogenated to give compounds of the general formula (X)

$R^3—CH_2—Hal$ (X)

in which $R^3$ has the abovementioned meaning, and
Hal represents halogen,
then reacted with acetate ions and hydrolysed to give compounds of the formula (XI)

$R^3$-$CH_2$-OH (XI)

in which
$R^3$ has the abovementioned meaning,
and these are then oxidized to compounds of the formula (II),
or in that
b) compounds of the general formula (XII)

$R^3$-COOR (XII)

in which
$R^3$ has the abovementioned meaning
and
R
- represents hydrogen or a $C_1$-$C_6$-alkyl radical,
are either directly reduced or compounds of the formula (XI) are first prepared by reduction and these are then oxidized.

**3.** Process for the production of medicaments, characterized in that at least one compound of the general formula I according to Claim 1 is converted into a suitable form for administration, optionally using customary auxiliaries and excipients.

**Revendications**

**Revendications pour les Etats contractants suivants : AT, BE, CH, DE, DK, FR, GB, GR, IT, LI, LU, NL, SE**

**1.** 4-quinolyl-dihydropyridines de formule générale (I)

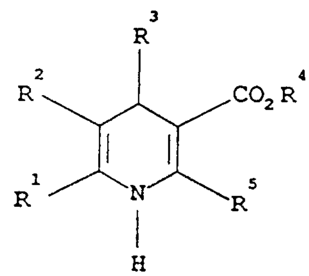

(I)

dans laquelle
$R^1$ et $R^5$ sont identiques ou différents et représentent un groupe alkyle linéaire ou ramifié ayant jusqu'à 6 atomes de carbone,
$R^2$ est un groupe nitro ou cyano, ou bien
$R^1$ et $R^2$ forment ensemble un noyau de lactone de formule

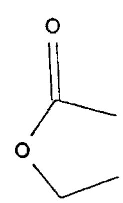

$R^3$ est un reste de formule

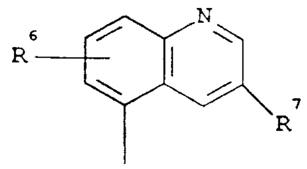

ou

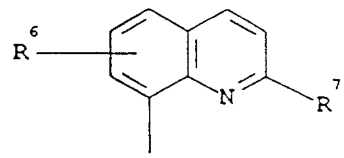

où

$R^6$ représente de l'hydrogène, du fluor, du chlore ou un reste alkyle ou alkoxy linéaire ou ramifié ayant chacun jusqu'à 2 atomes de carbone,

$R^7$ est un groupe phényle qui est substitué le cas échéant par du fluor, du chlore, un radical nitro, cyano, trifluorométhyle ou par un radical alkyle ou alkoxy linéaire ou ramifié ayant chacun jusqu'à 6 atomes de carbone, ou représente un groupe thiényle ou un groupe pyridyle qui sont monosubstitués le cas échéant par du fluor ou du chlore,

$R^4$ est de l'hydrogène ou
un groupe alkyle ou un groupe alcényle linéaire ou ramifié ayant chacun jusqu'à 8 atomes de carbone, ces deux groupes étant éventuellement substitués par du fluor, du chlore, un radical hydroxy, carboxy, cyano, nitro ou par un radical alkylthio, alkoxy, alkoxycarbonyle, acyle ou acyloxy linéaire ou ramifié ayant chacun jusqu'à 6 atomes de carbone ou par un radical phénoxy ou phényle

et leurs sels acceptables du point de vue physiologique.

**2.** Composés de formule générale I suivant la revendication 1, dans laquelle $R^1$, $R^2$, $R^1$ et $R^2$ ensemble, $R^5$ ainsi que $R^3$ ont la définition indiquée dans la revendication 1,

$R^6$ est de l'hydrogène, du chlore ou un radical méthyle,

$R^7$

- désigne un groupe phényle qui est substitué le cas échéant par du fluor, du chlore, un radical nitro, trifluorométhyle ou par un radical alkyle ou un radical alkoxy linéaire ou ramifié ayant chacun jusqu'à 4 atomes de carbone, ou bien
- un groupe pyridyle ou thiényle qui est monosubstitué le cas échéant par du fluor ou du chlore,

$R^4$ représente de l'hydrogène ou un groupe alkyle linéaire ou ramifié ayant jusqu'à 6 atomes de carbone, qui est substitué le cas échéant par un radical hydroxy, carboxy, cyano ou par un radical alkoxycarbonyle, alkoxy ou acyloxy linéaire ou ramifié ayant dans chaque cas jusqu'à 4 atomes de carbone.

**3.** Procédé de production de composés de formule générale I suivant la revendication 1, caractérisé en ce que lorsque $R^1$ et $R^2$ ont la définition indiquée ci-dessus mais ne forment pas ensemble un noyau de lactone,

[A] on fait réagir des composés de formule générale (II)

$R^3$-CHO (II)

dans laquelle

$R^3$ a la définition indiquée ci-dessus, tout d'abord avec des esters d'acide acétylacétique de formule générale (III)

$R^5-CO-CH_2-CO_2-R^4$ (III)

dans laquelle

$R^4$ et $R^5$ ont la définition indiquée ci-dessus, le cas échéant avec isolement des composés ylidéniques correspondants de formule générale (IV)

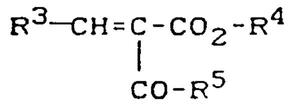 (IV)

dans laquelle
$R^3$, $R^4$ et $R^5$ ont la définition indiquée ci-dessus,
puis,
on fait réagir ces composés
avec des composés de formule (V)

$R^1$-CO-$CH_2$-$R^2$ (V)

dans laquelle $R^1$ et $R^2$ ont la définition indiquée ci-dessus,
en présence d'ammoniac ou de sels d'ammonium, ou directement avec des dérivés aminés de formule générale (VI)

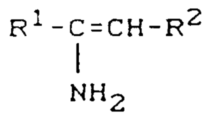 (VI)

dans laquelle
$R^1$ et $R^2$ ont la définition indiquée ci-dessus, le cas échéant en présence de solvants organiques,
ou bien

[B] on fait réagir les aldéhydes de formule générale (II) tout d'abord avec les composés de formule générale (V),
le cas échéant avec isolement des composés ylidéniques de formule générale (VII)

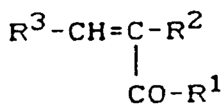 (VII)

dans laquelle
$R^1$, $R^2$ et $R^3$ ont la définition indiquée ci-dessus,
et dans une étape suivante, on les fait réagir avec les composés indiqués ci-dessus de formule générale (III) dans des solvants inertes, en présence d'ammoniac ou de sels d'ammonium ou directement avec des dérivés d'acides énaminocarboxyliques de formule générale (VIII)

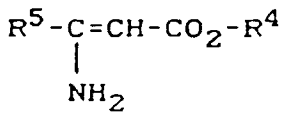 (VIII)

dans laquelle
$R^4$ et $R^5$ ont la définition indiquée ci-dessus, ou bien, au cas où $R^1$ et $R^2$ forment ensemble un noyau de lactone,
[C] on prépare tout d'abord par les procédés indiqués en [A] et [B] des composés de formule générale (Ia)

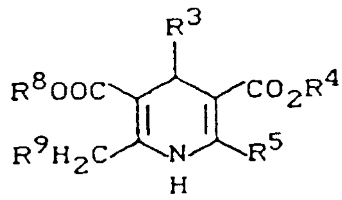

(Ia)

dans laquelle
$R^3$, $R^4$ et $R^5$ ont la définition indiquée ci-dessus,
$R^8$ représente un reste alkyle en $C_1$ à $C_6$ et
$R^9$ est un groupe partant classique
puis on effectue par des procédés connus une cyclisation catalysée par un acide ou par une base,
et au cas où $R^4$ ne représente pas de l'hydrogène,
[D] on fait réagir des composés de formule générale (I) dans laquelle $R^1$, $R^2$, $R^3$, $R^4$ et $R^5$ ont la définition indiquée ci-dessus et $R^4$ représente de l'hydrogène, le cas échéant en passant par un dérivé d'acide réactif, avec les alcools correspondants, et on obtient alors en utilisant les acides carboxyliques ($R^4$ = H) de pureté énantiomérique, les énantiomères correspondants des esters.

**4.** Aldéhydes de formule générale II

$R^3$—CHO (II)

dans laquelle
$R^3$ représente un reste de formule

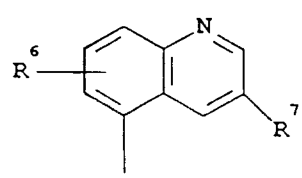

et $R^6$ et $R^7$ ont la même définition que dans la revendication 1.

**5.** Procédé de production d'aldéhydes de formule générale II suivant la revendication 4, caractérisé en ce que
a) on oxyde des composés de formule générale (IX)

$R^3$—$CH_3$ (III)

dans laquelle $R^3$ a la définition indiquée ci-dessus,
directement en composés de formule (II), ou bien on les halogène tout d'abord en composés de formule générale (X)

$R^3$—$CH_2$—Hal (X)

dans laquelle $R^3$ a la définition indiquée ci-dessus et Hal représente un halogène,
puis on les fait réagir avec des ions acétate et on les saponifie en composés de formule (XI)

$R^3$-$CH_2$-OH (XI)

dans laquelle
$R^3$ a la définition indiquée ci-dessus et on oxyde ensuite ces composés en composés de formule (II),
ou bien

b) on réduit des composés de formule générale (XII)

$R^3$-COOR (XII)

dans laquelle
$R^3$ a la définition indiquée ci-dessus
et
R est de l'hydrogène ou un reste alkyle en $C_1$ à $C_6$,
directement ou bien on prépare, par réduction, tout d'abord des composés de formule (XI) que l'on oxyde ensuite.

**6.** Médicament contenant au moins un composé de formule générale I suivant la revendication 1.

**7.** Procédé de production de médicaments, caractérisé en ce qu'on fait prendre une forme administrable appropriée à au moins un composé de formule générale I suivant la revendication 1, en utilisant le cas échéant des substances auxiliaires et des supports classiques.

**8.** Utilisation de composés de formule générale I suivant la revendication 1 dans la préparation de médicaments.

**Revendications pour l'Etat contractant suivant : ES**

**1.** Procédé de production de 4-quinolyl-dihydropyridines de formule générale I

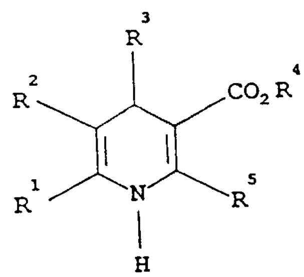

(I)

dans laquelle
$R^1$ et $R^5$ sont identiques ou différents et représentent un groupe alkyle linéaire ou ramifié ayant jusqu'à 6 atomes de carbone,
$R^2$ est un groupe nitro ou cyano, ou bien
$R^1$ et $R^2$ forment ensemble un noyau de lactone de formule

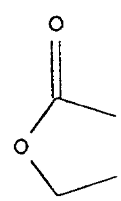

$R^3$ est un reste de formule

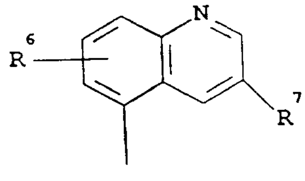

ou

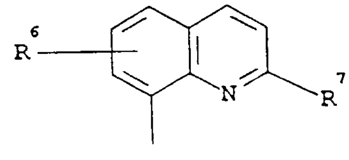

où

$R^6$ représente de l'hydrogène, du fluor, du chlore ou un reste alkyle ou alkoxy linéaire ou ramifié ayant chacun jusqu'à 2 atomes de carbone,

$R^7$ est un groupe phényle qui est substitué le cas échéant par du fluor, du chlore, un radical nitro, cyano, trifluorométhyle ou par un radical alkyle ou alkoxy linéaire ou ramifié ayant chacun jusqu'à 6 atomes de carbone, ou représente un groupe thiényle ou un groupe pyridyle qui sont monosubstitués le cas échéant par du fluor ou du chlore,

$R^4$ est de l'hydrogène ou un groupe alkyle ou un groupe alcényle linéaire ou ramifié ayant chacun jusqu'à 8 atomes de carbone, ces deux groupes étant éventuellement substitués par du fluor, du chlore, un radical hydroxy, carboxy, cyano, nitro ou par un radical alkylthio, alkoxy, alkoxycarbonyle, acyle ou acyloxy linéaire ou ramifié ayant chacun jusqu'à 6 atomes de carbone ou par un radical phénoxy ou phényle

et de leurs sels acceptables du point de vue physiologique, caractérisé en ce que lorsque $R^1$ et $R^2$ ont la définition indiquée ci-dessus mais ne forment pas ensemble un noyau de lactone,

[A] on fait réagir des composés de formule générale (II)

$R^3$-CHO (II)

dans laquelle

$R^3$ a la définition indiquée ci-dessus, tout d'abord avec des esters d'acide acétylacétique de formule générale (III)

$R^5-CO-CH_2-CO_2-R^4$ (III)

dans laquelle

$R^4$ et $R^5$ ont la définition indiquée ci-dessus, le cas échéant avec isolement des composés ylidéniques correspondants de formule générale (IV)

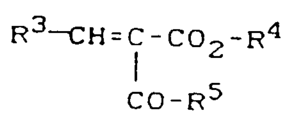

(IV)

dans laquelle

$R^3$, $R^4$ et $R^5$ ont la définition indiquée ci-dessus,

puis,

on fait réagir ces composés

avec des composés de formule (V)

$R^1-CO-CH_2-R^2$ (V)

dans laquelle $R^1$ et $R^2$ ont la définition indiquée ci-dessus,

en présence d'ammoniac ou de sels d'ammonium,

ou directement avec des dérivés aminés de formule générale (VI)

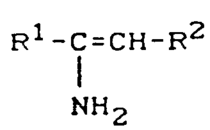

(VI)

dans laquelle

$R^1$ et $R^2$ ont la définition indiquée ci-dessus, le cas échéant en présence de solvants organiques,

ou bien

[B] on fait réagir les aldéhydes de formule générale (II) tout d'abord avec les composés de formule générale (V),
le cas échéant avec isolement des composés ylidéniques de formule générale (VII)

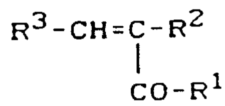

(VII)

dans laquelle
$R^1$, $R^2$ et $R^3$ ont la définition indiquée ci-dessus,
et dans une étape suivante, on les fait réagir avec les composés indiqués ci-dessus de formule générale (III) dans des solvants inertes, en présence d'ammoniac ou de sels d'ammonium ou directement avec des dérivés d'acides énaminocarboxyliques de formule générale (VIII)

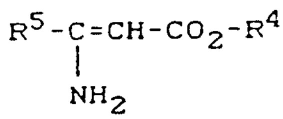

(VIII)

dans laquelle
$R^4$ et $R^5$ ont la définition indiquée ci-dessus, ou bien, au cas où $R^1$ et $R^2$ forment ensemble un noyau de lactone,
[C] on prépare tout d'abord par les procédés indiqués en [A] et [B] des composés de formule générale (Ia)

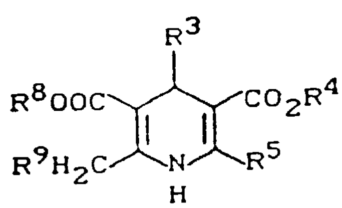

(Ia)

dans laquelle
$R^3$, $R^4$ et $R^5$ ont la définition indiquée ci-dessus,
$R^8$ représente un reste alkyle en $C_1$ à $C_6$
et
$R^9$ est un groupe partant classique,
puis on effectue par des procédés connus une cyclisation catalysée par un acide ou par une base,
et au cas où $R^4$ ne représente pas de l'hydrogène,
[D] on fait réagir des composés de formule générale (I) dans laquelle $R^1$, $R^2$, $R^3$, $R^4$ et $R^5$ ont la définition indiquée ci-dessus et $R^4$ représente de l'hydrogène, le cas échéant en passant par un dérivé d'acide réactif, avec les alcools correspondants, et on obtient alors en utilisant les acides carboxyliques ($R^4$ = H) de pureté énantiomérique, les énantiomères correspondants des esters.

**2.** Procédé de production d'aldéhydes de formule générale II

$R^3$—CHO (II)

dans laquelle
$R^3$ représente un reste de formule

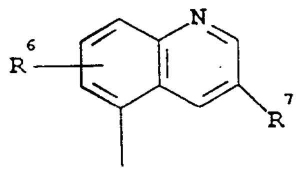

$R^6$ et $R^7$ ayant les mêmes définitions que dans la revendication 1,
caractérisé en ce que :

a) on oxyde des composés de formule générale (IX)

$R^3—CH_3$ (III)

dans laquelle $R^3$ a la définition indiquée ci-dessus,
directement en composés de formule (II), ou bien on les halogène tout d'abord en composés de formule générale (X)

$R^3—CH_2—Hal$ (X)

dans laquelle $R^3$ a la définition indiquée ci-dessus et Hal représente un halogène,
puis on les fait réagir avec des ions acétate et on les saponifie en composés de formule (XI)

$R^3\text{-}CH_2\text{-}OH$ (XI)

dans laquelle
$R^3$ a la définition indiquée ci-dessus et on oxyde ensuite ces composés en composés de formule (II),
ou bien

b) on réduit des composés de formule générale (XII)

$R^3\text{-}COOR$ (XII)

dans laquelle
$R^3$ a la définition indiquée ci-dessus
et
R est de l'hydrogène ou un reste alkyle en $C_1$ à $C_6$,
directement ou bien on prépare, par réduction, tout d'abord des composés de formule (XI) que l'on oxyde ensuite.

**3.** Procédé de préparation de médicaments, caractérisé en ce qu'on fait prendre une forme administrable appropriée à au moins un composé de formule générale I suivant la revendication 1, en utilisant éventuellement des substances auxiliaires et des supports classiques.